(19) Europäisches Patentamt
European Patent Office
Office européen des brevets

(11) Veröffentlichungsnummer: **0 296 316 B1**

(12) **EUROPÄISCHE PATENTSCHRIFT**

(45) Veröffentlichungstag der Patentschrift: **01.09.93**

(51) Int. Cl.$^5$: **C07D 211/90**, C07D 401/04, C07D 405/04, C07D 413/04, C07D 417/04, A61K 31/44

(21) Anmeldenummer: **88104691.6**

(22) Anmeldetag: **24.03.88**

Verbunden mit 88903212.4/0343193 (europäische Anmeldenummer/Veröffentlichungsnummer) durch Entscheidung vom 18.07.90.

Die Akte enthält technische Angaben, die nach dem Eingang der Anmeldung eingereicht wurden und die nicht in dieser Patentschrift enthalten sind.

(54) **1,4-Dihydropyridin-Enantiomere.**

(30) Priorität: **27.03.87 CH 1190/87**
**27.03.87 CH 1184/87**

(43) Veröffentlichungstag der Anmeldung:
**28.12.88 Patentblatt 88/52**

(45) Bekanntmachung des Hinweises auf die Patenterteilung:
**01.09.93 Patentblatt 93/35**

(84) Benannte Vertragsstaaten:
**AT BE CH DE ES FR GB GR IT LI LU NL SE**

(56) Entgegenhaltungen:
**EP-A- 0 172 509**
**EP-A- 0 174 653**
**EP-A- 0 176 956**
**EP-A- 0 240 828**
**EP-A- 0 242 829**
**EP-A- 0 244 595**
**WO-A-86/03748**
**DE-A- 3 627 742**
**GB-A- 2 142 021**

**Progress in Pharmacology, vol. 5/1, 1982, Seite 25**

(73) Patentinhaber: **Byk Gulden Lomberg Chemische Fabrik GmbH**
**Postfach 10 03 10**
**D-78403 Konstanz(DE)**

(72) Erfinder: **Amschler, Hermann, Dr.**
**Hohenhewenstrasse 19**
**D-7760 Radolfzell(DE)**
Erfinder: **Eistetter, Klaus, Dr.**
**Säntisblick 7**
**D-7750 Konstanz 19(DE)**
Erfinder: **Eltze, Manfrid, Dr.**
**Schützenstrasse 20**
**D-7750 Konstanz(DE)**
Erfinder: **Flockerzi, Dieter, Dr.**
**Ackerweg 26**
**D-7753 Allensbach(DE)**
Erfinder: **Klemm, Kurt, Prof. Dr.**
**Im Weinberg 2**
**D-7753 Allensbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

Erfinder: **Kohl, Bernhard, Dr.**
**Heinrich-v.-Tettingen-Strasse 35a**
**D-7750 Konstanz(DE)**
Erfinder: **Kolassa, Norbert, Prof. Dr.**
**Lerchenweg 12**
**D-7750 Konstanz(DE)**
Erfinder: **Sanders, Karl, Dr.**
**Felchengang 12**
**D-7750 Konstanz(DE)**
Erfinder: **Schudt, Christian, Dr.**
**Hoheneggstrasse 112**
**D-7750 Konstanz(DE)**
Erfinder: **Ulrich, Wolf-Rüdiger, Dr.**
**Hebelstrasse 3**
**D-7750 Konstanz(DE)**

## Beschreibung

Die Erfindung betrifft neue Enantiomere, Verfahren zu ihrer Herstellung, ihre Verwendung und sie enthaltende Arzneimittel. Die erfindungsgemäßen Verbindungen werden in der pharmazeutischen Industrie zur Herstellung von Arzneimitteln eingesetzt.

### Bekannter technischer Hintergrund

Es ist bekannt, daß bestimmte, in 4-Position substituierte 1,4-Dihydropyridinderivate pharmakologisch nützliche Eigenschaften aufweisen. Ferner ist bekannt, daß diese 1,4-Dihydropyridinderivate - sofern sie in den Positionen 2 und 6 und/oder in den Positionen 3 und 5 unterschiedlich (unsymmetrisch) substituiert sind - in der Position 4 ein Chiralitätszentrum aufweisen. Außerdem ist bekannt, daß die pharmakologischen Eigenschaften der 1,4-Dihydropyridine von der absoluten Konfiguration in der 4-Position beeinflußt werden können [siehe hierzu sowie zu allgemeinen Struktur-/Wirkungsbetrachtungen R. Mannhold et al., Progr. Pharmacol. 5, 25 (1982)]. Aufgrund dieser unterschiedlichen pharmakologischen Eigenschaften wurde schon verschiedentlich versucht, enantiomer reine 1,4-Dihydropyridine herzustellen [siehe z.B. DE-OS 2935451, DE-OS 3320616, EP-A2 0166296, EP-A 172 509, Shibaruma et al. Chem. Pharm.Bull. 28, 2809 (1980)]. Mit den bisher bekannten Verfahren lassen sich jedoch entweder nicht alle denkbaren Enantiomeren bekannter 1,4-Dihydropyridine herstellen, oder diese Verfahren liefern die enantiomer reinen 1,4-Dihydropyridine nur in geringen Ausbeuten oder in einer ungenügenden Reinheit.

### Beschreibung der Erfindung

Überraschenderweise wurde nun ein neues Verfahren zur Herstellung enantiomer reiner, optisch aktiver 1,4-Dihydropyridinen gefunden, das die gewünschten Enantiomere in guter Ausbeute und hoher Reinheit liefert. Mit diesem Verfahren lassen sich neue Enantiomere bekannter 1,4-Dihydropyridine herstellen.

Gegenstand der Erfindung sind somit in einem ersten Aspekt enantiomer reine Dihydropyridine der Formel I

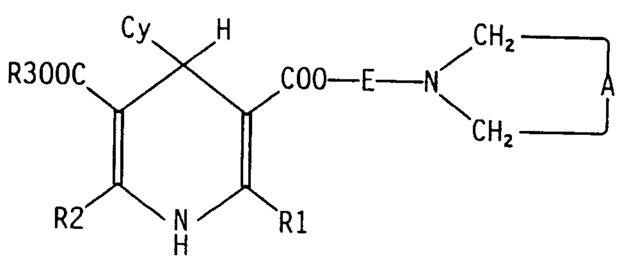

(I),

die in der 4-Position des Dihydropyridins die gleiche Konfiguration aufweisen, wie das als Vorprodukt einsetzbare (+)-1-Ethoxymethyl-1,4-dihydro-5-methoxycarbonyl-2,6-dimethyl-4-(3-nitrophenyl)-pyridin-3-carbonsäure/Cinchoninsalz, das das linear polarisierte Licht der Wellenlänge 589 nm mit $[\alpha]_D^{22}$ = + 101,5° (c = 1, Chloroform) dreht,
und worin

| | |
|---|---|
| Cy | 3-Nitrophenyl oder 2,3-Dichlorphenyl bedeutet, |
| R1 und R2 | gleich oder verschieden sind und 1-4C-Alkyl bedeuten, |
| R3 | 1-4C-Alkyl bedeutet, |
| E | Ethylen oder Propylen bedeutet, |
| A | $-CH_2-C(R6)R7-CH_2-$ oder $-CH_2-NR8-CH_2-$ bedeutet, |
| R6 | Wasserstoff (H) oder Phenyl bedeutet, |
| R7 | Phenyl bedeutet, |
| R8 | Aryl oder Benzhydryl bedeutet, wobei |

Aryl für einen Ring der Formel

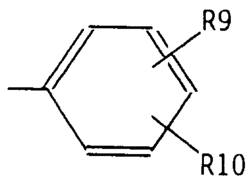

steht, worin R9 und R10 gleich oder verschieden sind und die Bedeutung Wasserstoff (H), 1-4C-Alkoxy oder Halogen haben,
und die Salze dieser Verbindungen.

Halogen im Sinne der Erfindung bedeutet Brom, Fluor und insbesondere Chlor. 1-4C-Alkyl ist geradkettig oder verzweigt und bedeutet beispielsweise einen Butyl-, i-Butyl-, sec.-Butyl-, t-Butyl-, Propyl-, Isopropyl-, Ethyl-oder insbesondere Methylrest.

1-4C-Alkoxy enthält neben dem Sauerstoffatom einen der vorstehend genannten 1-4C-Alkylreste. Bevorzugt sind der Methoxy- und der Ethoxyrest.

Als Salze kommen alle Salze mit Säuren in Betracht. Besonders erwähnt seien die pharmakologisch verträglichen Salze der in der pharmazeutischen Industrie üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Metembonat, Stearat, Tosilat, 2-Hydroxy-3-naphthoat, 3-Hydroxy-2-naphthoat oder Mesilat, aber auch Salze mit Bumetanid, Furosemid, Azosemid, Galosemid, Besunid, Piretanid, Etacrynsäure, Tienilinsäure oder 4-Chlor-sulfamoyl-benzoesäure.

Gegenstand der Erfindung sind somit in einem ersten Aspekt neue, erstmals isolierte bzw. isolierbare enantiomerenreine 1,4-Dihydropyridine der Formel I.

Eine Ausgestaltung (Ausgestaltung a) der Erfindung sind enantiomer reine Verbindungen der Formel I, worin Cy, R1, R2, R3 und E die eingangs genannten Bedeutungen haben,

A $-CH_2-C(R6)R7-CH_2-$ bedeutet,
R6 Wasserstoff (H) bedeutet und
R7 Phenyl bedeutet,

und die Salze dieser Verbindungen.

Die Ausgestaltung a kann charakterisiert werden durch die folgende Formel Ia

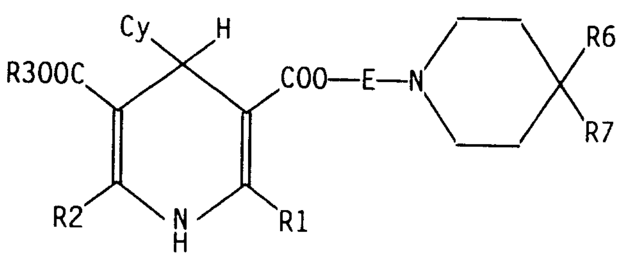

(Ia)

worin die Substituenten und Symbole die für die Ausgestaltung a genannten Bedeutungen haben.

Hervorzuhebende Verbindungen der Ausgestaltung a sind solche der Formel Ia, worin

Cy 3-Nitrophenyl oder 2,3-Dichlorphenyl bedeutet,
R1 Methyl oder Ethyl bedeutet,
R2 Methyl oder Ethyl bedeutet,
R3 Methyl oder Ethyl bedeutet,
E Ethylen oder Propylen bedeutet,
R6 Wasserstoff bedeutet und
R7 Phenyl bedeutet,

und ihre Salze.

Besonders bevorzugte Verbindungen der Ausgestaltung a sind solche der Formel Ia, worin
Cy 3-Nitrophenyl bedeutet,
R1 Methyl bedeutet,
R2 Methyl bedeutet,
R3 Methyl bedeutet,
E Ethylen oder Propylen bedeutet,
R6 Wasserstoff bedeutet und
R7 Phenyl bedeutet,
und ihre Salze.

Als beispielhafte, besonders erwähnenswerte Verbindungen der Ausgestaltung a seien genannt:
(R)-1,4-Dihydro-2,6-dimethyl-4-(2,3-dichlorphenyl)-pyridin-3,5-dicarbonsäure-3-methyl-5-[2-(4-phenylpiperidyl-1)-ethyl]-ester,
(R)-1,4-Dihydro-2,6-dimethyl-4-(2,3-dichlorphenyl)-pyridin-3,5-dicarbonsäure-3-methyl-5-[3-(4-phenylpiperidyl-1)-propyl]-ester,
(R)-1,4-Dihydro-2,6-dimethyl-4-(3-nitrophenyl)-pyridin-3,5-dicarbonsäure-3-methyl-5-[2-(4-phenylpiperidyl-1)-ethyl]-ester,
(R)-1,4-Dihydro-2,6-dimethyl-4-(3-nitrophenyl)-pyridin-3,5-dicarbonsäure-3-methyl-5-[3-(4-phenylpiperidyl-1)-propyl]-ester,
und ihre Salze.

Eine weitere Ausgestaltung (Ausgestaltung b) der Erfindung sind enantiomer reine Verbindungen der Formel I, worin Cy, R1, R2, R3 und E die eingangs genannten Bedeutungen haben,
A $-CH_2-NR8-CH_2-$ bedeutet,
R8 Aryl oder Benzhydryl bedeutet, wobei Aryl die eingangs angegebenen Bedeutungen hat,
und die Salze dieser Verbindungen.

Die Ausgestaltung b kann charakterisiert werden durch die folgende Formel Ib

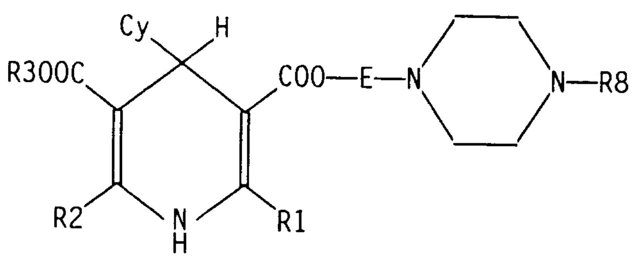
(Ib)

worin die Substituenten und Symbole die für die Ausgestaltung b genannten Bedeutungen haben.

Hervorzuhebende Verbindungen der Ausgestaltung b sind solche der Formel Ib, worin
Cy 3-Nitrophenyl oder 2,3-Dichlorphenyl bedeutet,
R1 Methyl oder Ethyl bedeutet,
R2 Methyl oder Ethyl bedeutet,
R3 Methyl oder Ethyl bedeutet,
E Ethylen oder Propylen bedeutet und
R8 Phenyl, 2-Methoxyphenyl, 2-Ethoxyphenyl, 2-Ethoxy-4-fluorphenyl, 3-Methoxyphenyl oder Benzhydryl bedeutet,
und ihre Salze.

Besonders bevorzugte Verbindungen der Ausgestaltung b sind solche der Formel Ib, worin
Cy 3-Nitrophenyl bedeutet,
R1 Methyl bedeutet,
R2 Methyl bedeutet,
R3 Methyl bedeutet,
E Ethylen oder Propylen bedeutet und
R8 Phenyl, 2-Methoxyphenyl, 2-Ethoxyphenyl, 2-Ethoxy-4-fluorphenyl oder Benzhydryl bedeutet,
und ihre Salze.

Als beispielhafte, besonders erwähnenswerte Verbindungen der Ausgestaltung b seien genannt:
(R)-1,4-Dihydro-2,6-dimethyl-4-(2,3-dichlorphenyl)-pyridin-3,5-dicarbonsäure-3-methyl-5-{2-[4-(2-methoxyphenyl)-1-piperazinyl]-ethyl}-ester,
(R)-1,4-Dihydro-2,6-dimethyl-4-(2,3-dichlorphenyl)-pyridin-3,5-dicarbonsäure-3-methyl-5-{3-[4-(2-methoxyphenyl)-1-piperazinyl]-propyl}-ester,
(R)-1,4-Dihydro-2,6-dimethyl-4-(2,3-dichlorphenyl)-pyridin-3,5-dicarbonsäure-3-methyl-5-[2-(4-benzhydryl-1-piperazinyl)-ethyl]-ester,
(R)-1,4-Dihydro-2,6-dimethyl-4-(2,3-dichlorphenyl)-pyridin-3,5-dicarbonsäure-3-methyl-5-[3-(4-benzhydryl-1-piperazinyl)-propyl]-ester,
(R)-1,4-Dihydro-2,6-dimethyl-4-(3-nitrophenyl)-pyridin-3,5-dicarbonsäure-3-methyl-5-{2-[4-(2-methoxyphenyl)-1-piperazinyl]-ethyl}-ester,
(R)-1,4-Dihydro-2,6-dimethyl-4-(3-nitrophenyl)-pyridin-3,5-dicarbonsäure-3-methyl-5-{3-[4-(2-methoxyphenyl)-1-piperazinyl]-propyl}-ester,
(R)-1,4-Dihydro-2,6-dimethyl-4-(3-nitrophenyl)-pyridin-3,5-dicarbonsäure-3-methyl-5-[2-(4-phenyl-1-piperazinyl)-ethyl]-ester,
(R)-1,4-Dihydro-2,6-dimethyl-4-(3-nitrophenyl)-pyridin-3,5-dicarbonsäure-3-methyl-5-[3-(4-phenyl-1-piperazinyl)-propyl]-ester,
(R)-1,4-Dihydro-2,6-dimethyl-4-(3-nitrophenyl)-pyridin-3,5-dicarbonsäure-3-methyl-5-[2-(4-benzhydryl-1-piperazinyl)-ethyl]-ester,
(R)-1,4-Dihydro-2,6-dimethyl-4-(3-nitrophenyl)-pyridin-3,5-dicarbonsäure-3-methyl-5-[3-(4-benzhydryl-1-piperazinyl)-propyl]-ester
(R)-1,4-Dihydro-2,6-dimethyl-4-(3-nitrophenyl)-pyridin-3,5-dicarbonsäure-3-methyl-5-{3-[4-(2-ethoxyphenyl)-1-piperazinyl]-propyl}-ester,
(R)-1,4-Dihydro-2,6-dimethyl-4-(3-nitrophenyl)-pyridin-3,5-dicarbonsäure-3-methyl-5-{2-[4-(2-ethoxyphenyl)-1-piperazinyl]-ethyl}-ester
(R)-1,4-Dihydro-2,6-dimethyl-4-(3-nitrophenyl)-pyridin-3,5-dicarbonsäure-3-methy-5-{3-[4-(3-methoxyphenyl)-1-piperazinyl]-propyl}-ester
(R)-1,4-Dihydro-2,6-dimethyl-4-(3-nitrophenyl)-pyridin-3,5-dicarbonsäure-3-methy-5-{2-[4-(3-methoxyphenyl)-1-piperazinyl]-ethyl}-ester
und ihre Salze.

Eine weitere Ausgestaltung (Ausgestaltung c) der Erfindung sind enantiomer reine Verbindungen der Formel I, worin

| | |
|---|---|
| Cy, R1, R2, R3 und E | die eingangs genannten Bedeutungen haben, |
| A | $-CH_2-C(R6)R7-CH_2-$ bedeutet, |
| R6 | Phenyl bedeutet und |
| R7 | Phenyl bedeutet, |

und die Salze dieser Verbindungen.

Die Ausgestaltung c kann charakterisiert werden durch die folgende Formel Ic

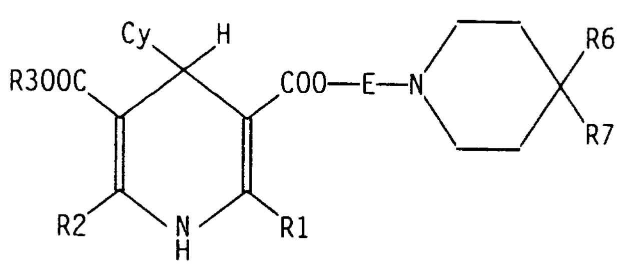

(Ic)

worin die Substituenten und Symbole die für die Ausgestaltung c genannten Bedeutungen haben.

Hervorzuhebende Verbindungen der Ausgestaltung c sind solche der Formel Ic, worin

| | |
|---|---|
| Cy | 3-Nitrophenyl oder 2,3-Dichlorphenyl bedeutet, |
| R1 | Methyl oder Ethyl bedeutet, |
| R2 | Methyl oder Ethyl bedeutet, |
| R3 | Methyl oder Ethyl bedeutet, |
| E | Ethylen oder Propylen bedeutet, |
| R6 | Phenyl bedeutet und |
| R7 | Phenyl bedeutet, |

und ihre Salze.

Besonders bevorzugte Verbindungen der Ausgestaltung c sind solche der Formel Ic, worin

| | |
|---|---|
| Cy | 3-Nitrophenyl bedeutet, |
| R1 | Methyl bedeutet, |

R2 Methyl bedeutet,
R3 Methyl oder Ethyl bedeutet,
E Ethylen oder Propylen bedeutet,
R6 Phenyl und
R7 Phenyl bedeutet,
und ihre Salze.

Als beispielhafte, besonders erwähnenswerte Verbindungen der Ausgestaltung c seien genannt:
(R)-1,4-Dihydro-2,6-dimethyl-4-(2,3-dichlorphenyl)-pyridin-3,5-dicarbonsäure-3-methyl-5-[2-(4,4-diphenylpiperidyl-1)-ethyl]-ester,
(R)-1,4-Dihydro-2,6-dimethyl-4-(2,3-dichlorphenyl)-pyridin-3,5-dicarbonsäure-3-methyl-5-[3-(4,4-diphenylpiperidyl-1)-propyl]-ester,
(R)-1,4-Dihydro-2,6-dimethyl-4-(3-nitrophenyl)-pyridin-3,5-dicarbonsäure-3-methyl-5-[2-(4,4-diphenylpiperidyl-1)-ethyl]-ester,
(R)-(+)-1,4-Dihydro-2,6-dimethyl-4-(3-nitrophenyl)-pyridin-3,5-dicarbonsäure-3-methyl-5-[3-(4,4-diphenyl-1-piperidinyl-1)-propyl]ester,
und ihre Salze.

Weiterer Gegenstand der Erfindung ist ein Verfahren zur Herstellung der Verbindungen der Formel I und ihrer Salze.

Das Verfahren ist dadurch gekennzeichnet, daß man enantiomer reine N-geschützte Dihydropyridincarbonsäuren der Formel II,

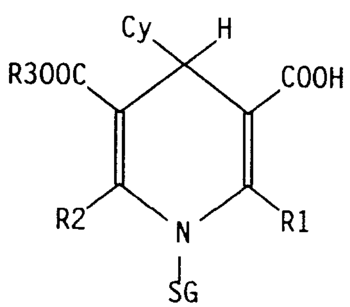

(II)

die in der 4-Position des Dihydropyridins die gleiche Konfiguration aufweisen, wie das als Vorprodukt einsetzbare (+)-1-Ethoxymethyl-1,4-dihydro-5-methoxycarbonyl-2,6-dimethyl-4-(3-nitrophenyl)-pyridin-3-carbonsäure/Cinchoninsalz, das das linear polarisierte Licht der Wellenlänge 589 nm mit $[\alpha]_D^{22} = +101{,}5°$ (c = 1, Chloroform) dreht, und worin R1, R2, R3 und Cy die vorstehend angegebenen Bedeutungen haben und SG eine Schutzgruppe darstellt,
mit einem omega-Dihalogenalkan Hal-E-Hal, worin Hal Halogen bedeutet und E die vorstehend angegebene Bedeutung hat, umsetzt und nach Abspaltung der Schutzgruppe SG den erhaltenen Halogenalkylester III

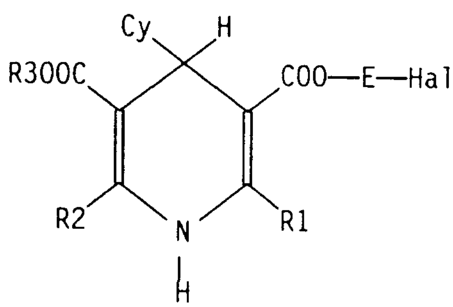

(III)

mit einem Amin der Formel IV oder einem Salz davon

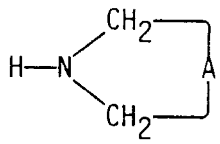

(IV)

zum Endprodukt I umsetzt und gewünschtenfalls anschließend erhaltene Salze der Verbindung I in die freien Basen oder die Verbindungen I in ihre Salze überführt.

Die Umsetzung von II mit omega-Dihalogenalkanen erfolgt bevorzugt unter basischen Bedingungen in Gegenwart eines Phasentransferkatalysators.

Als omega-Dihalogenalkane sind bevorzugt 1,2-Dihalogenethane und 1,3-Dihalogenpropane, insbesondere 1,2-Dibromethan und 1,3-Dibrompropan zu nennen.

Als Phasentransferkatalysatoren seien neben Oniumsalzen, wie z.B. Tetrabutylammoniumbromid oder Benzyltriethylammoniumchlorid, vor allem Kronenether, wie Dibenzo-[18]krone-6, Dicyclohexyl-[18]krone-6 und insbesondere [18]Krone-6 erwähnt.

Als eingesetzte Basen, die wenigstens im molaren Verhältnis, vorzugsweise im Überschuß eingesetzt werden, kommen anorganische Basen, wie Alkalimetallhydroxide (z.B. Natrium- oder Kaliumhydroxid), oder insbesondere Alkalimetallcarbonate (z.B. Natrium- oder vorzugsweise Kaliumcarbonat) in Frage. Beim Arbeiten in einem wasserfreien Lösungsmittel werden die verwendeten Hydroxide bzw. Carbonate vorzugsweise in feingepulverter Form eingesetzt.

Die Umsetzung erfolgt (je nach Art des Phasentransferkatalysators und der eingesetzten Base) in wasserhaltigen oder wasserfreien organischen Lösungsmitteln, oder in einem Gemisch aus Wasser und einem mit Waser nicht oder kaum mischbaren organischen Lösungsmittel. Als Wasser/Lösungsmittelmischungen seien beispielsweise die Mischungen von Wasser mit Chloroform, Dichlormethan oder Benzol genannt. Als wasserhaltige oder wasserfreie Lösungsmittel seien beispielsweise Dichlormethan, Acetonitril oder Aceton genannt.

Die in den Beispielen genannten Lösungsmittel, Basen und Phasentransferkatalysatorenstellen nur eine exemplarische Auswahl dar. Welche weiteren Kombinationen von Lösungsmitteln, Basen und Phasentransferkatalysatoren noch geeignet sind, ist dem Fachmann aufgrund seines Fachwissens geläufig.

Die Wahl der Reaktionstemperatur bei der Umsetzung von II mit Dihalogenalkanen hängt von den übrigen Reaktionsbedingungen ab, wobei in der Regel Temperaturen zwischen 20 °C und der Siedetemperatur des eingesetzten Lösungsmittels bevorzugt sind.

Als Schutzgruppen SG kommen vor allem solche Gruppen in Frage, die in das der Verbindung II zugrundeliegende Vorprodukt leicht und in hohen Ausbeuten eingeführt werden können, die bei der Umsetzung von II mit Dihalogenalkanen keine Nebenreaktionen eingehen und die am Ende glatt wieder abgespalten werden können. Als bevorzugte Schutzgruppen SG seien beispielsweise Alkoxymethylgruppen oder Benzyloxymethylgruppen, insbesondere die Ethoxymethylgruppe genannt. Die Abspaltung der Schutzgruppe erfolgt in saurem Medium, beispielsweise in 1N Salzsäure oder vorzugsweise in wasserfreier Ameisensäure, unter Reaktionsbedingungen, wie sie dem Fachmann geläufig sind. Erfindungsgemäß kann die Abspaltung der Schutzgruppe auch nach der Umsetzung mit dem Amin IV erfolgen.

Die Umsetzung der Halogenalkylester III mit Aminen IV erfolgt auf eine Weise, wie sie für die Umsetzung von Alkylhalogeniden mit sekundären Aminen dem Fachmann vertraut ist.

Die Umsetzung wird in geeigneten, vorzugsweise inerten organischen Lösungsmitteln in Gegenwart von Wasser oder ohne Wasser durchgeführt. Beispielsweise seien genannt Ether, wie Dioxan, Diethylether, Tetrahydrofuran, Glykolmonoethylether oder Glykoldimethylether; Ketone, wie Aceton oder Ethylmethylketon; aromatische Kohlenwasserstoffe, wie Xylol oder Toluol; oder chlorierte Kohlenwasserstoffe, wie Methylenchlorid, Chloroform, Tetrachlorethylen oder Dichlorethan; oder polare aprotische Lösungsmittel wie Dimethylformamid oder Dimethylsulfoxid.

Die Reaktionstemperaturen können - je nach Reaktivität der Edukte - in einem weiten Bereich variieren. Im allgemeinen wird die Umsetzung bei Temperaturen zwischen 20 °C und 150 °C, vorzugsweise zwischen 20 °C und 100 °C, insbesondere bei der Siedetemperatur des verwendeten Lösungsmittels durchgeführt.

Das Verfahren kann bei Normaldruck oder bei erhöhtem Druck durchgeführt werden, wobei das Arbeiten bei Normaldruck die Regel ist. Die Reaktion wird in Gegenwart einer Base (z.B. eines anorganischen Carbonates, wie Kaliumcarbonat) oder unter Einsetzung eines Überschusses an Amin IV durchgeführt.

Die Isolierung und Reinigung der erhaltenen erfindungsgemäßen Verbindungen I erfolgt in an sich bekannter Weise z. B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft. Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), oder einem offenkettigen oder cyclischen Ether, wie Dioxan oder Tetrahydrofuran, das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Ausgestaltungen des Verfahrens sind solche, bei denen die Substituenten und Symbole die für die Verbindungen der Ausgestaltungen a, b und c genannten Bedeutungen haben. Hervorzuhebende, bevorzugte und besonders bevorzugte Verfahren sind solche, bei denen die Substituenten und Symbole die für die hervorzuhebenden, bevorzugten und besonders bevorzugten Verbindungen genannten Bedeutungen haben.

Von besonderer Bedeutung ist in diesem Zusammenhang die Anwendung des erfindungsgemäßen Verfahrens zur Herstellung des das linear polarisierte Licht der Wellenlänge 589 nm in (+)-Richtung drehenden Enantiomers des 1,4-Dihydro-2,6-dimethyl-4-(3-nitrophenyl)-pyridin-3,5-dicarbonsäure-3-methyl-5-[3-(4,4-diphenylpiperidyl-1)propyl]-esters, also des (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-esters, und seiner Salze.

Ohne Berücksichtigung der absoluten Konfiguration in der 4-Position des 1,4-Dihydropyridins wird die erfindungsgemäß hergestellte Verbindung durch die folgende Formel I* beschrieben:

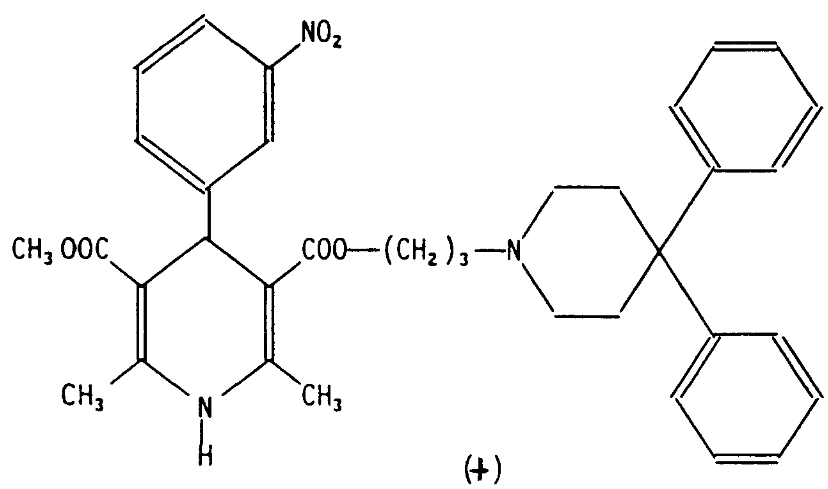

(I*)

Das Verfahren ist in diesem Fall dadurch gekennzeichnet, daß man eine N-geschützte Dihydropyridincarbonsäure der Formel II*

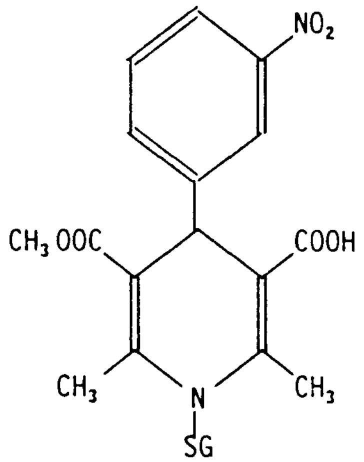

(II*)

(+)

mit 1,3-Dibrompropan umsetzt und nach Abspaltung der Schutzgruppe SG den erhaltenen Brompropylester III*

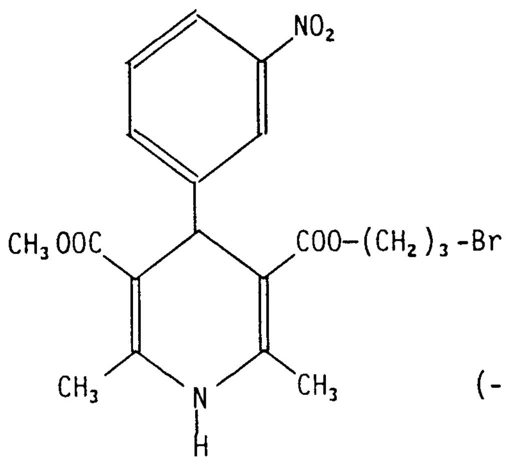

(III*)

(-)

mit Diphenylpiperidin oder einem Salz davon zum Endprodukt I* umsetzt und gewünschtenfalls anschließend ein erhaltenes Salz der Verbindung I* in die freie Base oder die Verbindung I* in ein Salz überführt.

Die enantiomer reinen Verbindungen der Formel II bzw. II* sind teils bekannt [Chem. Pharm. Bull. 28(9) 2809-2812 (1980)], teils neu. Sie können auf bekannte Weise [Chem. Pharm. Bull. 28(9) 2809-2812 (1980)] oder nach einem neuen Verfahren hergestellt werden.

Das neue Verfahren ist dadurch gekennzeichnet, daß man in Haloethylester der Formel V (Hal = Chlor, insbesondere Brom)

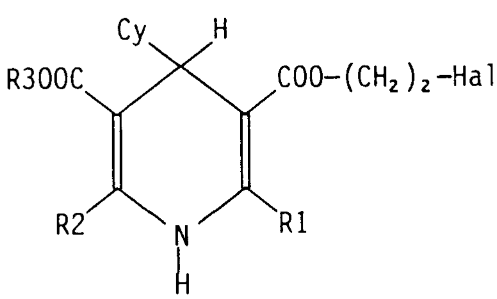

(V)

die Schutzgruppe SG einführt, in dem so erhaltenen, N-geschützten Haloethylester der Formel VI

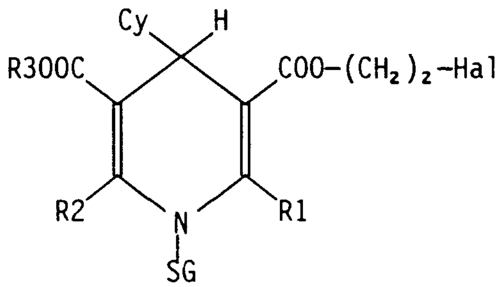

(VI)

Hal gegen ein als Akzeptor (= Akz) fungierendes Nucleophil austauscht, aus dem erhaltenen Akzeptorethylester der Formel VII

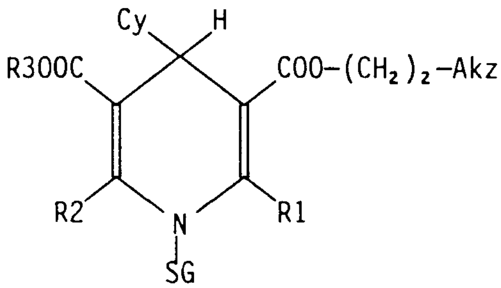

(VII)

unter basischen Bedingungen den Akzeptorethylrest abspaltet und das so erhaltene Racemat der Verbindung II in die Enantiomeren spaltet.

Zur Einführung der Schutzgruppe SG wird zweckmäßigerweise so vorgegangen, daß der Haloethylester V in 1-Stellung deprotoniert und anschließend mit einer Verbindung SG-X umgesetzt wird, wobei X für eine geeignete Fluchtgruppe steht.

Als Deprotonierungsmittel kommen vor allem solche Agenzien in Frage, für die die Acidität des Protons am Stickstoff groß genug ist um eine Anionbildung zu erzielen. Neben metallorganischen Verbindungen, wie z.B. Butyllithium, seien vorzugsweise Metallhydride, insbesondere das Natriumhydrid erwähnt. Die Fluchtgruppe X der Verbindung SG-X ist eine Gruppe, die bei der Umsetzung von SG-X mit dem deprotonierten V leicht abgespalten wird. Falls die Schutzgruppe SG eine Alkoxymethylgruppe ist, ist X vorzugsweise ein Halogenatom, insbesondere ein Chloratom.

Die Deprotonierung und anschließende Einführung der Schutzgruppe wird in inerten, wasserfreien Lösungsmitteln vorgenommen, wie sie für das Arbeiten mit starken Deprotonierungsmitteln geeignet sind. Beispielsweise seien offenkettige oder cyclische Ether, wie Diethylether, Dioxan oder Tetrahydrofuran genannt. Die Umsetzung erfolgt bevorzugt unter schonenden Reaktionsbedingungen bei Temperaturen um oder unter 0 °C.

Als Akzeptorreste bei der weiteren Umsetzung kommen insbesondere solche Reste in Frage, die einen mesomer elektronenanziehenden Effekt ausüben können. Beispielsweise seien die Nitrogruppe oder die Azidogruppe, bevorzugt die Nitrilgruppe oder eine substituierte Sulfonylgruppe genannt.

Der Halogenaustausch im N-geschützten Haloethylester der Formel VI erfolgt vorzugsweise durch Umsetzung mit Salzen deren Anionen geeignet sind, als kovalent gebundene Akzeptorsubstituenten zu fungieren. Bevorzugt ist die Umsetzung mit anorganischen Cyaniden, insbesondere mit Kaliumcyanid oder Natriumcyanid, oder die Umsetzung mit Alkalisulfinaten, insbesondere mit Natrium-4-toluolsulfinat, gewünschtenfalls unter Zusatz katalytischer Mengen eines quartären Ammoniumsalzes, wie z.B. Tetrabutylammoniumcyanid oder Benzyltriethylammoniumchlorid, in inerten, vorzugsweise aprotischen, polaren Lösungsmitteln wie beispielsweise Aceton, Dimethylsulfoxid oder Dimethylformamid, bei Temperaturen, die bevorzugt zwischen 0 °C und 50 °C liegen.

Die sich an den Halogenaustausch anschließende Abspaltung des Akzeptor-ethylrestes unter basischen Bedingungen ist eine dem Fachmann geläufige Reaktion, wie sie z.B. in der DE-OS 2847237 beschrieben ist. Die zunächst als Racemat anfallende Verbindung II kann mit Hilfe enantiomer reiner, optisch aktiver Basen in üblicher Weise über die diastereomeren Salze in die Enantiomeren (+)-II und (-)-II getrennt

werden [siehe z.B. Chem. Pharm. Bull. 28, 2809 (1980)] [1]. Als enantiomer reine optisch aktive Basen seien insbesondere Cinchonidin und Cinchonin genannt.

Die als Ausgangsverbindungen eingesetzten Haloethylester V sind z.B. aus der DE-OS 2847237 bekannt.

Die folgenden Herstellungsbeispiele sollen die Erfindung näher erläutern. Schmp. bedeutet Schmelzpunkt, h steht für Stunden, Kp. steht für Siedepunkt, Zers. bedeutet Zersetzung. Unter Ether wird Diethylether verstanden.

## Beispiele

### Endprodukte

1. (-)-1,4-Dihydro-2,6-dimethyl-4-(3-nitrophenyl)-pyridin-3,5-dicarbonsäure-3-methyl-5-[3-(4,4-diphenyl-1-piperidinyl)-propyl]ester-hydrochlorid

86,6 g (-)-1,4-Dihydro-2,6-dimethyl-4-(3-nitrophenyl)-pyridin-3,5-dicarbonsäure-3-methyl-5-(3-brompropyl)-ester, 50 g 4,4-Diphenylpiperidin-hydrochlorid und 69 g feingemahlenes Kaliumcarbonat werden zusammen in 300 ml Dimethylformamid unter kräftigem Rühren und unter einer Stickstoffathmosphäre 5 h auf 100 °C erhitzt. Nach Abkühlen werden nacheinander 500 ml Ethylacetat und 1 l Wasser zugegeben und kräftig ausgerührt. Nach Phasentrennung wird die organische Phase noch viermal mit Wasser gewaschen, über Natriumsulfat getrocknet und im Vakuum eingeengt. Der Rückstand wird in 1 l Dioxan gelöst und die Lösung unter Kühlen mit 15,2 ml konzentrierter Salzsäurelösung (12,5 M, d = 1,19) versetzt; dann werden im Vakuum ca. 200 ml des Lösungsmittelgemisches abdestilliert. Beim Stehen bei Raumtemperatur kristallisiert das Produkt spontan oder nach Animpfen oder Anreiben; es wird nach 16 h abgesaugt, mit Dioxan und Diisopropylether gewaschen und im Vakuum bei 80-100 °C getrocknet. Zur Reinigung wird das Rohprodukt in Dichlormethan gelöst. Nach Zugabe von 800 ml Dioxan wird das Dichlormethan wieder abdestilliert. Das nach Animpfen und 16-stündigem Stehen bei Raumtemperatur auskristallisierte Produkt wird abgesaugt, mit Dioxan und Diisopropylether gewaschen und bei 100 °C getrocknet. Man erhält 97 g der Titelverbindung vom Schmp. 158-160 °C mit

$$[\alpha]^{22}_{436} = -39^{0}$$

(c = 1, Methanol) bzw. $[\alpha]^{22}_{D}$ = -14,4 ° (c = 1, Methanol).

### Ausgangsverbindungen

A. (-)-1,4-Dihydro-2,6-dimethyl-4-(3-nitrophenyl)-pyridin-3,5-dicarbonsäure-3-methyl-5-(3-brompropyl)-ester

168,5 g (+)-1-Ethoxymethyl-1,4-dihydro-5-methoxycarbonyl-2,6-dimethyl-4(3-nitrophenyl)-pyridin-3-carbonsäure-Cinchoninsalz {$[\alpha]^{22}_{D}$ = + 101,5 ° (c = 1, Chloroform)} (hergestellt aus (±)1-Ethoxymethyl-1,4-dihydro-5-methoxycarbonyl-2,6-dimethyl-4-(3-nitrophenyl)-pyridin-3-carbonsäure und Cinchonin analog Beispeil B) werden in 1,5 l Dichlormethan gelöst und dann unter Kühlung und kräftigem Rühren mit 1,2 l 0,2 N Salzsäurelösung versetzt. Durch Zugabe von 2 N Salzsäurelösung wird in der wässerigen Phase ein stabiler pH von 2 eingestellt. Nach Phasentrennung wird die organische Phase noch viermal mit Salzsäurelösung von pH 2 und dann mit Wasser gewaschen, über Natriumsulfat getrocknet und eingeengt. Der ölige Rückstand wird in 1,1 l Aceton gelöst und mit 375 g feinvermahlenem Kaliumcarbonat, 375 ml 1,3-Dibrompropan und 1,2 g [18] Krone-6 versetzt. Das Gemisch wird 24 h kräftig bei Raumtemperatur gerührt, dann wird abgesaugt und der Filterkuchen mit Aceton gewaschen. Das Filtrat wird am Rotationsverdampfer bei schwachem Vakuum eingeengt und dann das überschüssige 1,3-Dibrompropan bei 0,02 mbar abdestilliert (Badtemperatur bis 45 °C). Der ölige Rückstand wird unter Eiskühlung mit 690 ml konzentrierter Ameisensäure übergossen, dann wird bei Raumtemperatur solange gerührt, bis eine klare Lösung entstanden ist (ca. 15 Min). Die Ameisensäure wird im Vakuum abdestilliert. Nach zweimaliger Zugabe und Abdestillieren von je 200 ml Toluol wird der Rückstand in 900 ml Dichlormethan gelöst. Die Lösung wird mit Natriumhydrogencarbonatlösung ausgerührt, mit Wasser gewaschen, über Natriumsulfat getrocknet und im Vakuum eingeengt. Das nach Zugabe von Diisopropylether spontan kristallisierende Produkt wird abgesaugt, mit Diisopropylether gewaschen und im Vakuum getrocknet. Man erhält 102 g der Titelverbindung vom Schmp. 112-114 °C und $[\alpha]_D^{22}$ = -13,8 ° (c = 1, Methanol).

B. (-)-1-Ethoxymethyl-1,4-dihydro-5-methoxycarbonyl-2,6-dimethyl-4-(3-nitrophenyl)-pyridin-3-carbonsäure-/Cinchonidinsalz

229,7 g (±)-1-Ethoxymethyl-1,4-dihydro-5-methoxycarbonyl-2,6-dimethyl-4--(3-nitrophenyl)-pyridin-3-carbonsäure und 173,2 g Cinchonidin werden in 2,4 l Ethanol in der Hitze gelöst. Bei langsamer Abkühlung kristallisiert die Titelverbindung aus und wird nach 2-tägigem Stehen bei Raumtemperatur abgesaugt und mit Ethanol gewaschen. Nach zweimaligem Umkristallisieren aus Ethanol erhält man 148 g der Titelverbindung vom Schmp. 185-185,5 °C und $[\alpha]_D^{22}$ = -63,4 ° (c = 1, Chloroform). Durch Aufarbeiten der Mutterlauge aus der 1. Kristallisation kann das entsprechende (+)-Enantiomere erhalten werden:
Die Mutterlauge wird im Vakuum zur Trockne eingedampft, der Rückstand in 2 l Chloroform gelöst und nach Zugabe von 1 l Wasser unter kräftigem Rühren durch Zutropfen von 2N Salzsäure ein stabiler pH von 2 bis 3 eingestellt. Die Phasen werden getrennt, die organische Phase noch so lange mit 0,01N Salzsäure ausgeschüttelt bis kein Cinchonidin mehr nachweisbar ist (Dünnschichtchromatographie), mit Wasser gewaschen und getrocknet. Nach Abdestillierer des Lösungsmittels mit Vakuum wird der Rückstand im Hochvakuum getrocknet und 85,6 g davon zusammen mit 54,5 g Cinchonin in ca. 2,4 l siedendem Ethanol gelöst. Das nach 3-tägigem Stehen bei Raumtemperatur auskristallisierte Produkt wird abgesaugt, mit Ethanol gewaschen und nochmals aus Ethanol umkristallisiert. Man erhält 88,5 g (+)-1-Ethoxymethyl-1,4-dihydro-5-methoxycarbonyl-2,6-dimethyl-4-(3-nitrophenyl)-pyridin-3-carbonsäure/Cinchoninsalz vom Schmp. 197 - 198 °C und $[\alpha]_D^{22}$ = +101,5 ° (c = 1, Methanol).

C. (±)-1-Ethoxymethyl-1,4-dihydro-5-methoxycarbonyl-2,6-dimethyl-4-(3-nitrophenyl)-pyridin-3-carbonsäure

a) 195,5 g (±)-1-Ethoxymethyl-1,4-dihydro-2,6-dimethyl-4-(3-nitrophenyl)-pyridin-3,5-dicarbonsäure-3-methyl-5-(2-bromethyl)-ester werden in 900 ml Dimethylformamid gelöst und mit 40,6 g gepulvertem Natriumcyanid und 0,5 g Tetrabutylammoniumcyanid versetzt. Das Gemisch wird 26 h bei Raumtemperatur gerührt, wobei nach ca. 4 h ein dicker Niederschlag ausfällt, dann werden 200 ml 2N Natriumhydroxid-Lösung zugetropft und 5 h bei Raumtemperatur weitergerührt. Das Gemisch wird in 4,5 l Wasser eingerührt und unter Kühlung und starkem Rühren mit 2N Salzsäurelösung bis pH 2,5 tropfenweise versetzt. Das dabei ausgefallene Produkt wird scharf abgesaugt, mit Wasser neutral gewaschen, über Nacht luftgetrocknet und dann in 400 ml Ethanol auf dem Dampfbad erhitzt (keine Lösung). Es wird 12 h bei Raumtemperatur stehengelassen, dann gekühlt, abgesaugt und getrocknet. Man erhält 142 g der Titelverbindung vom Schmp. 176-176,5 °C (Zersetzung).
b) 5 g (±)-1-Ethoxymethyl-1,4-dihydro-2,6-dimethyl-4-(3-nitrophenyl)-pyridin-3,5-dicarbonsäure-3-methyl-5-(2-bromethyl)-ester werden in 35 ml Dimethylformamid gelöst, 3,55 g (20 mMol) 4-Toluolsulfinsäure-Natrium-Salz und 0,1 g Benzyltriethylammoniumchlorid zugesetzt und 20 h bei 20 °C gerührt. Man gibt 11 ml 2N Natronlauge zu, rührt 5 h bei 20 °C, weitere 3 h bei 80 °C und gießt nach Abkühlung auf 120 ml Eiswasser. Nach Zugabe von Essigsäure bis pH 3,8 wird 3 h im Eisbad gerührt, vom ausgefallenen Feststoff filtriert, mit Wasser neutral gewaschen und getrocknet. Das Rohprodukt wird in 10 ml Ethanol ausgekocht, abgekühlt, filtriert und getrocknet. Man erhält 3,31 g der Titelverbindung vom Schmp. 176-176,5 °C (Zers.).

D. (±)-1-Ethoxymethyl-1,4-dihydro-2,6-dimethyl-4-(3-nitrophenyl)-pyridin-3,5-dicarbonsäure-3-methyl-5-(2-bromethyl)-ester

10,65 g einer 80 %igen Suspension von Natriumhydrid in Paraffinöl werden in 300 ml wasserfreiem Tetrahydrofuran aufgeschlämmt. Bei -5 bis -7 °C wird unter Rühren eine Lösung von 120 g (±)-1,4-Dihydro-2,6-dimethyl-4-(3-nitrophenyl)-pyridin-3,5-dicarbonsäure-3-methyl-5-(2-bromethyl)-ester und 28,4 g Chlormethylethylether in 300 ml wasserfreiem Tetrahydrofuran langsam innerhalb von 4 h zugetropft. Nach einer weiteren Stunde Rühren bei 0 °C werden nacheinander 600 ml Toluol und 210 ml Wasser zugegeben. Die Phasen werden getrennt, die organische Phase wird mit Wasser gewaschen, über Nacht getrocknet und im Vakuum eingeengt. Der Rückstand wird in 390 ml warmem Ethanol gelöst, dann wird unter Rühren abgekühlt. Dabei kristallisiert das Produkt aus. Es wird auf 0 °C gekühlt, abgesaugt, mit eiskaltem Ethanol gewaschen und getrocknet. Man erhält 98,5 g der Titelverbindung vom Schmp. 72,5-74 °C.

E. (±)-1,4-Dihydro-2,6-dimethyl-4-(3-nitrophenyl)-pyridin-3,5-dicarbonsäure-3-methylester-5-(2-bromethyl)-ester

494 g 2-(3-Nitrobenzyliden)-acetessigsäure-(2-bromethyl)-ester und 173,5 g 3-Aminocrotonsäuremethylester werden unter Rühren und unter einer Stickstoffatmosphäre in 1750 ml Isopropanol bei 75-80 °C gelöst. Das Gemisch wird danach sehr langsam im Heizbad abgekühlt. Nach Animpfen mit der Titelverbindung wird 16 h bei Raumtemperatur gerührt, dann gekühlt, abgesaugt, mit eiskaltem Ether gewaschen und getrocknet. Man erhält 481 g der Titelverbindung vom Schmp. 139-141 °C.

F. 2-(3-Nitrobenzyliden)-acetessigsäure-(2-bromethyl)-ester

405 g Acetessigsäure-2-bromethylester werden in 1500 ml Isopropanol gelöst. Unter Rühren werden 293 g 3-Nitrobenzaldehyd, 4,6 g Essigsäure und 6,8 g Piperidin zugegeben. Das Gemisch wird bei 40 °C so lange gerührt bis eine klare Lösung entstanden ist. Man läßt langsam abkühlen, impft mit einigen Kristallen der Titelverbindung an und rührt bei Raumtemperatur 48 h lang, dann wird gekühlt, abgesaugt, mit kaltem Isopropanol gewaschen und im Vakuum bei 50 °C getrocknet. Man erhält 495 g der Titelverbindung vom Schmp. 94,5-95,5 °C.

G. Acetessigsäure-2-bromethylester

250 g 2-Bromethanol werden in 1,3 l Dichlormethan gelöst und mit 1,2 g 4-Dimethylaminopyridin versetzt. Unter kräftigem Rühren werden 400 ml einer 50%igen Diketenlösung in Aceton so zugetropft, daß das Lösungsmittel mäßig siedet. Nach dem Zutropfen wird noch 1 h bei Siedetemperatur gerührt und dann über Nacht stehengelassen. Nach Abdampfen des Lösungsmittels am Rotationsverdampfer im Vakuum wird der Rückstand destilliert. Man erhält 402 g der Titelverbindung mit Kp. 80-83 °C/0,04 mbar.

Gewerbliche Anwendbarkeit

Die durch das erfindungsgemäße Verfahren hergestellten enantiomer reinen Verbindungen der Formel I und ihre Salze besitzen wertvolle Eigenschaften, die sie gewerblich verwertbar machen. Sie stellen beispielsweise wirksame Vasodilatoren mit coronartherapeutischen Eigenschaften dar. Darüberhinaus besitzen die erfindungsgemäßen Verbindungen hemmende Wirkung auf den Calciumeinstrom sowie fördernde Wirkung auf den Kaliumausstrom von Zellen, glattmuskulär relaxierende und peripher, coronar, cerebral und renal gefäßerweiternde sowie salidiuretische, antithrombotische, antiarteriosklerotische und günstige hämorheologische Eigenschaften.

Durch Bereitstellung der in der Hauptwirkung besonders wirksamen Enantiomeren wird einerseits die Belastung durch ihre weniger wirksamen Stereoisomeren, die teilweise sogar entgegengesetzte Wirkung oder verstärkte Nebenwirkungen zeigen können, deutlich herabgemindert. Andererseits können die in der obengenannten Hauptwirkung schwächer wirksamen Enantiomeren für andere Therapiezwecke eingesetzt werden, die bisher für diese Stoffklasse - aufgrund der in den Racematen überwiegenden Hauptwirkung - nicht zugänglich waren.

Die ausgezeichnete Wirksamkeit der erfindungsgemäßen Verbindungen der Formel I und ihrer Salze gestattet ihren Einsatz in der Humanmedizin, wobei als Indikation beispielsweise koronare Herzkrankheiten (Koronarinsuffizienz, Angina Pectoris, Myocardinfarkt etc.), periphere und cerebrale Zirkulationsstörungen (Gehirnschlag, temporäre cerebrale Durchblutungsstörungen, Migräne, Schwindel, renale Arterienverengung etc.), hypertrophe Kardiomyopathie, Herzinsuffizienz, Krankheiten, die auf einer erhöhten Wasser- und Natriumretention beruhen und Krankheiten, die auf einem erhöhten Calciumeinstrom beruhen, wie z.B. Spasmen glattmuskulärer Organe (Atemwege, Gastrointestinaltrakt, Urogenitaltrakt etc.) sowie Arrhythmie, Arteriosklerose und Zellschädigungen verschiedener Genese (z.B. Hypoxie) in Betracht kommen.

Ein weiterer Gegenstand der Erfindung ist daher ein Verfahren zur Behandlung von Säugetieren, insbesondere Menschen, die an einer der obengenannten Krankheiten erkrankt sind. Das Verfahren ist dadurch gekennzeichnet, daß man dem erkrankten Individuum eine therapeutisch wirksame und pharmakologisch verträgliche Menge einer oder mehrerer Verbindungen der Formel I verabreicht.

Gegenstand der Erfindung sind außerdem die Verbindungen der Formel I zur Anwendung bei der Behandlung der genannten Krankheiten.

Ebenso umfaßt die Erfindung die Verwendung von Verbindungen der Formel I bei der Herstellung von Arzneimitteln, die zur Bekämpfung der genannten Krankheiten eingesetzt werden.

Ein weiterer Gegenstand der Erfindung sind Arzneimittel, die eine oder mehrere Verbindungen der allgemeinen Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragees, Kapseln, Suppositorien, Pflastern (z.B. als TTS), Emulsionen, Suspensionen, Aerosolen, Sprays, Salben, Cremes, Gelen oder Lösungen eingesetzt, wobei der Wirkstoffgehalt vorteilhafterweise zwischen 0,1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, rektal, per inhalationem oder parenteral (insbesondere perlingual, intravenös oder percutan) appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 10, vorzugsweise 0,05 bis 5 mg/kg Körpergewicht, gewünschtenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Bei einschleichender Dosierung wird zu Beginn der Behandlung eine geringere Dosis verabreicht, dann langsam auf eine höhere Dosis übergegangen. Nach Erreichen des gewünschten Therapieerfolges wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der genannten Krankheiten eingesetzt werden, so können die pharmazeutischen Zubereitungen auch einen oder mehrere andere pharmakologisch aktive Bestandteile anderer Arzneimittelgruppen, wie andere Vasodilatoren, Antihypertensiva, alpha-1-Rezeptorenblocker, alpha-2-Rezeptorstimulatoren, beta-1-Rezeptorenblokker, beta-2-Rezeptorstimulatoren, ACE-Hemmstoffe, Nitroverbindungen, Cardiotonika, Diuretika, Saluretika, Alkaloide, Analgetika, Lipidsenker, Antikoagulantien, Anticholinergika, Methylxanthine, Antiarrhythmika, Antihistaminika, Dopaminstimulatoren, Serotonin-Rezeptorenblocker etc., wie Nifedipin, Dihydralazin, Prazosin, Clonidin, Atenolol, Labetalol, Fenoterol, Captopril, Isosorbiddinitrat, Digoxin, Milrinon, Mefrusid, Clopamid, Spironolacton, Chlorthalidon, Furosemid, Polythiazid, Hydrochlorothiazid, Reserpin, Dihydroergocristin, Rescinnamin, Rauwolfia-Gesamtalkaloide, Acetylsalicylsäure, Bezafibrat, Warfarin, Atropin, Theophyllin, Lidocain, Astemizol, Bromocryptin, Ketanserin etc. enthalten.

## Patentansprüche

### Patentansprüche für folgende Vertragsstaaten : AT, BE, CH, DE, FR, GB, IT, LI, LU, NL, SE

**1.** Enantiomer reine Dihydropyridine der Formel I,

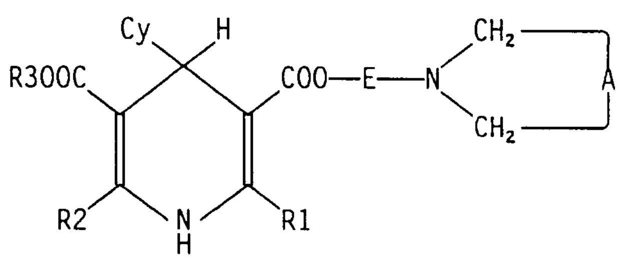

(I),

die in der 4-Position des Dihydropyridins die gleiche Konfiguration aufweisen, wie das als Vorprodukt einsetzbare (+)-1-Ethoxymethyl-1,4-dihydro-5-methoxycarbonyl-2,6-dimethyl-4-(3-nitrophenyl)-pyridin-3-carbonsäure/Cinchoninsalz, das das linear polarisierte Licht der Wellenlänge 589 nm mit $[\alpha]_D^{22}$ = + 101,5° (c = 1, Chloroform) dreht,

und worin

Cy 3-Nitrophenyl oder 2,3-Dichlorphenyl bedeutet,

| | |
|---|---|
| R1 und R2 | gleich oder verschieden sind und 1-4C-Alkyl bedeuten, |
| R3 | 1-4C-Alkyl bedeutet, |
| E | Ethylen oder Propylen bedeutet, |
| A | $-CH_2-C(R6)R7-CH_2-$ oder $-CH_2-NR8-CH_2-$ bedeutet, |
| R6 | Wasserstoff (H) oder Phenyl bedeutet, |
| R7 | Phenyl bedeutet, |
| R8 | Aryl oder Benzhydryl bedeutet, wobei |

Aryl für einen Ring der Formel

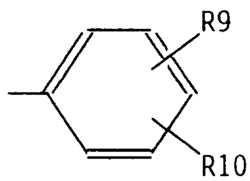

steht, worin R9 und R10 gleich oder verschieden sind und die Bedeutung Wasserstoff (H), 1-4C-Alkoxy oder Halogen haben, und die Salze dieser Verbindungen.

**2.** Enantiomer reine Verbindungen nach Anspruch 1, gekennzeichnet durch die Formel Ia

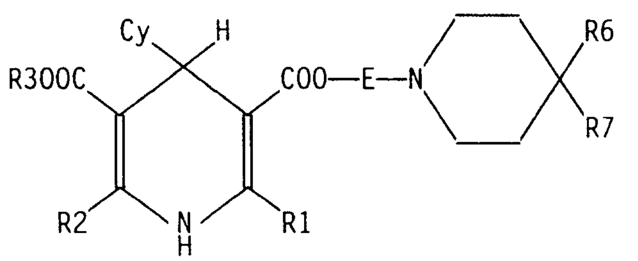

(Ia)

worin

| | |
|---|---|
| Cy | 3-Nitrophenyl oder 2,3-Dichlorphenyl bedeutet, |
| R1 | Methyl oder Ethyl bedeutet, |
| R2 | Methyl oder Ethyl bedeutet, |
| R3 | Methyl oder Ethyl bedeutet, |
| E | Ethylen oder Propylen bedeutet, |
| R6 | Wasserstoff bedeutet und |
| R7 | Phenyl bedeutet, |

und ihre Salze.

**3.** Enantiomer reine Verbindungen nach Anspruch 1, gekennzeichnet durch die Formel Ib

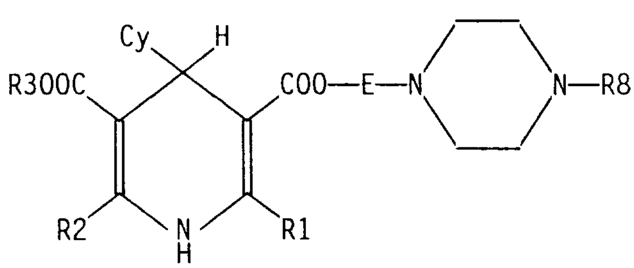

(Ib)

worin

| | |
|---|---|
| Cy | 3-Nitrophenyl oder 2,3-Dichlorphenyl bedeutet, |

R1 Methyl oder Ethyl bedeutet,
R2 Methyl oder Ethyl bedeutet,
R3 Methyl oder Ethyl bedeutet,
E Ethylen oder Propylen bedeutet und
R8 Phenyl, 2-Methoxyphenyl, 2-Ethoxyphenyl, 2-Ethoxy-4-fluorphenyl, 3-Methoxyphenyl oder Benzhydryl bedeutet,

und ihre Salze.

**4.** Enantiomer reine Verbindungen nach Anspruch 1, gekennzeichnet durch die Formel Ic

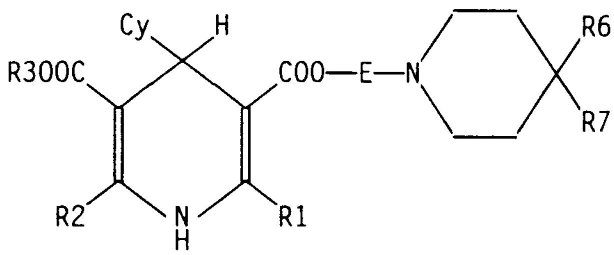

(Ic)

worin

Cy 3-Nitrophenyl oder 2,3-Dichlorphenyl bedeutet,
R1 Methyl oder Ethyl bedeutet,
R2 Methyl oder Ethyl bedeutet,
R3 Methyl oder Ethyl bedeutet,
E Ethylen oder Propylen bedeutet,
R6 Phenyl bedeutet und
R7 Phenyl bedeutet,

und ihre Salze.

**5.** (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und seine Salze,

**6.** Verfahren zur Herstellung der enantiomer reinen Verbindungen der Formel I nach Anspruch 1, und ihren Salzen, dadurch gekennzeichnet, daß man enantiomer reine N-geschützte Dihydropyridincarbonsäuren der Formel II,

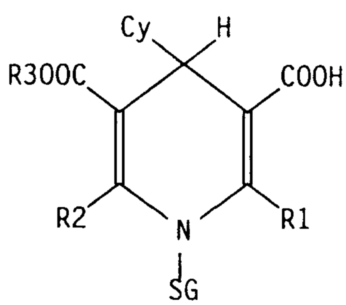

(II)

die in der 4-Position des Dihydropyridins die gleiche Konfiguration aufweisen, wie das als Vorprodukt einsetzbare (+)-1-Ethoxymethyl-1,4-dihydro-5-methoxycarbonyl-2,6-dimethyl-4-(3-nitrophenyl)-pyridin-3-carbonsäure/Cinchoninsalz, das das linear polarisierte Licht der Wellenlänge 589 nm mit $[\alpha]_D^{22}$ = +101,5° (c = 1, Chloroform) dreht, und worin R1, R2, R3 und Cy die in Anspruch 1 angegebenen Bedeutungen haben und SG eine Schutzgruppe darstellt,
mit einem omega-Dihalogenalkan Hal-E-Hal, worin Hal Halogen bedeutet und E die in Anspruch 1 angegebene Bedeutung hat, umsetzt und nach Abspaltung der Schutzgruppe SG den erhaltenen Halogenalkylester III

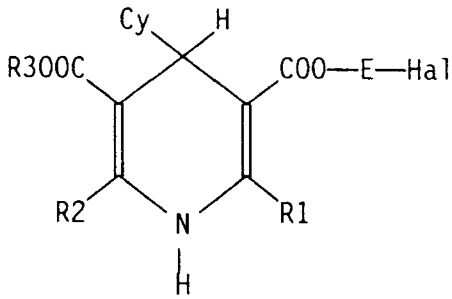

(III)

mit einem Amin der Formel IV oder einem Salz davon

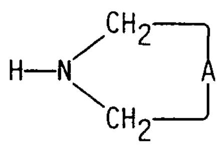

(IV)

worin A die in Anspruch 1 angegebene Bedeutung hat, umsetzt und gewünschtenfalls anschließend erhaltene Salze der Verbindung I in die freien Basen oder die Verbindungen I in ihre Salze überführt.

**7.** Verfahren nach Anspruch 6 zur Herstellung des (+)-1,4-Dihydro-2,6-dimethyl-4-(3-nitrophenyl)-pyridin-3,5-dicarbonsäure-3-methyl-5-[3-(4,4-diphenylpiperidyl-1)-propyl]-esters der Formel I* und seiner Salze,

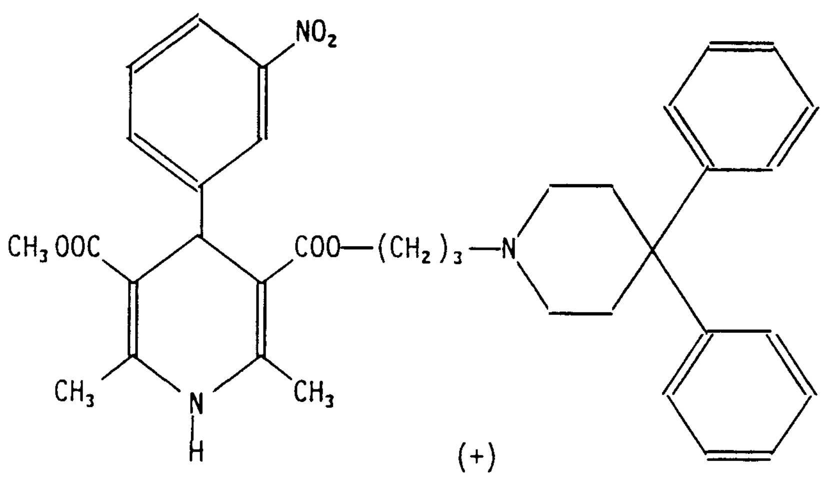

(+)

(I*)

dadurch gekennzeichnet, daß man eine N-geschützte Dihydropyridincarbonsäure der Formel II*

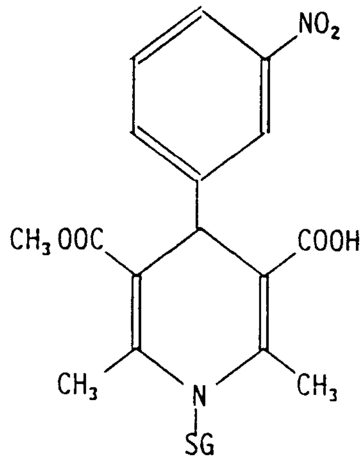

(II*)

(+)

mit 1,3-Dibrompropan umsetzt und nach Abspaltung der Schutzgruppe SG den erhaltenen Brompropylester III*

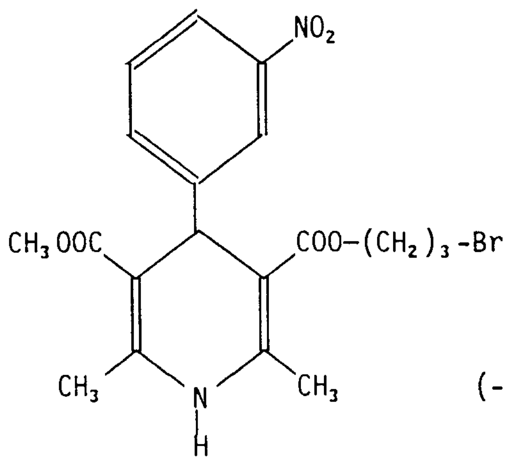

(III*)

(-)

mit Diphenylpiperidin oder einem Salz davon umsetzt und gewünschtenfalls anschließend ein erhaltenes Salz der Verbindung I* in die freie Base oder die Verbindung I* in ein Salz überführt.

**8.** Verfahren nach Anspruch 6 oder 7, dadurch gekennzeichnet, daß die Schutzgruppe SG eine Alkoxymethylgruppe oder Benzyloxymethylgruppe ist.

**9.** Arzneimittel enthaltend eine oder mehrere Verbindungen nach einem oder mehreren der Ansprüche 1 bis 5 und/oder ihre pharmakologisch verträglichen Salze.

**10.** Verbindungen nach einem oder mehreren der Ansprüche 1 bis 5 und ihre pharmakologisch verträglichen Salze zur Anwendung bei der Behandlung und/oder Prophylaxe der Arteriosklerose.

**Patentansprüche für folgende Vertragsstaaten : ES, GR**

**1.** Verfahren zur Herstellung von enantiomer reinen Dihydropyridinen der Formel I,

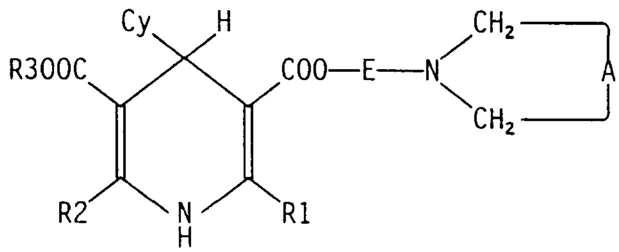

(I),

die in der 4-Position des Dihydropyridins die gleiche Konfiguration aufweisen, wie das als Vorprodukt einsetzbare (+)-1-Ethoxymethyl-1,4-dihydro-5-methoxycarbonyl-2,6-dimethyl-4-(3-nitrophenyl)-pyridin-3-carbonsäure/Cinchoninsalz, das das linear polarisierte Licht der Wellenlänge 589 nm mit $[\alpha]_D^{22}$ = + 101,5° (c = 1, Chloroform) dreht,
und worin

| | |
|---|---|
| Cy | 3-Nitrophenyl oder 2,3-Dichlorphenyl bedeutet, |
| R1 und R2 | gleich oder verschieden sind und 1-4C-Alkyl bedeuten, |
| R3 | 1-4C-Alkyl bedeutet, |
| E | Ethylen oder Propylen bedeutet, |
| A | $-CH_2-C(R6)R7-CH_2-$ oder $-CH_2-NR8-CH_2-$ bedeutet, |
| R6 | Wasserstoff (H) oder Phenyl bedeutet, |
| R7 | Phenyl bedeutet, |
| R8 | Aryl oder Benzhydryl bedeutet, wobei |

Aryl für einen Ring der Formel

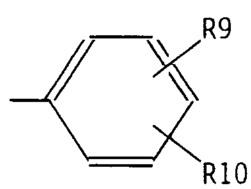

steht, worin R9 und R10 gleich oder verschieden sind und die Bedeutung Wasserstoff (H), 1-4C-Alkoxy oder Halogen haben,
und ihren Salzen, dadurch gekennzeichnet, daß man enantiomer reine N-geschützte Dihydropyridincarbonsäuren der Formel II,

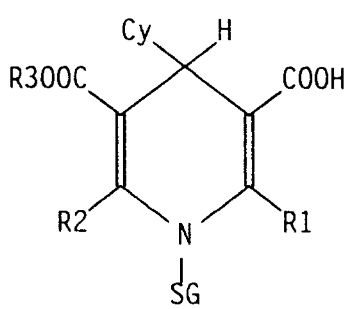

(II)

die in der 4-Position des Dihydropyridins die gleiche Konfiguration aufweisen, wie das als Vorprodukt einsetzbare (+)-1-Ethoxymethyl-1,4-dihydro-5-methoxycarbonyl-2,6-dimethyl-4-(3-nitrophenyl)-pyridin-3-carbonsäure/Cinchoninsalz, das das linear polarisierte Licht der Wellenlänge 589 nm mit $[\alpha]_D^{22}$ = + 101,5° (c = 1, Chloroform) dreht, und worin R1, R2, R3 und Cy die oben angegebenen Bedeutungen

haben und SG eine Schutzgruppe darstellt, mit einem omega-Dihalogenalkan Hal-E-Hal, worin Hal Halogen bedeutet und E die vorstehend angegebene Bedeutung hat, umsetzt und nach Abspaltung der Schutzgruppe SG den erhaltenen Halogenalkylester III

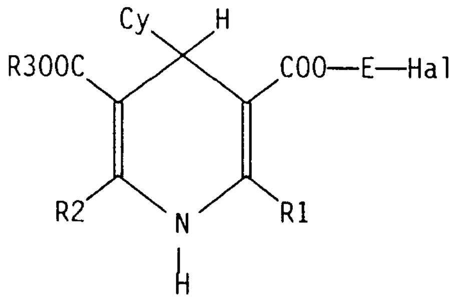

(III)

mit einem Amin der Formel IV oder einem Salz davon

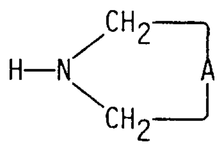

(IV)

worin A die oben angegebene Bedeutung hat, umsetzt und gewünschtenfalls anschließend erhaltene Salze der Verbindung I in die freien Basen oder die Verbindungen I in ihre Salze überführt.

**2.** Verfahren nach Anspruch 1, dadurch gekennzeichnet, daß Verbindungen der Formel Ia

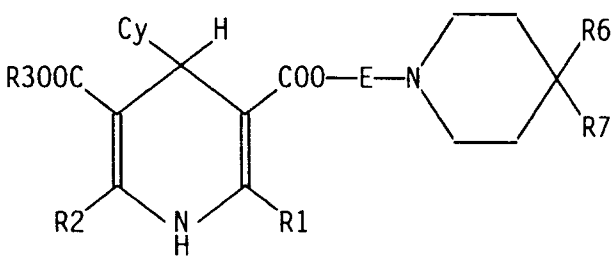

(Ia)

worin

| | |
|---|---|
| Cy | 3-Nitrophenyl oder 2,3-Dichlorphenyl bedeutet, |
| R1 | Methyl oder Ethyl bedeutet, |
| R2 | Methyl oder Ethyl bedeutet, |
| R3 | Methyl oder Ethyl bedeutet, |
| E | Ethylen oder Propylen bedeutet, |
| R6 | Wasserstoff bedeutet und |
| R7 | Phenyl bedeutet, |

und ihre Salze hergestellt werden.

**3.** Verfahren nach Anspruch 1, dadurch gekennzeichnet, daß Verbindungen der Formel Ib

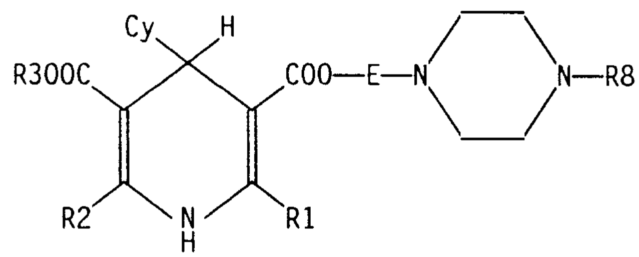

(Ib)

worin

| | |
|---|---|
| Cy | 3-Nitrophenyl oder 2,3-Dichlorphenyl bedeutet, |
| R1 | Methyl oder Ethyl bedeutet, |
| R2 | Methyl oder Ethyl bedeutet, |
| R3 | Methyl oder Ethyl bedeutet, |
| E | Ethylen oder Propylen bedeutet und |
| R8 | Phenyl, 2-Methoxyphenyl, 2-Ethoxyphenyl, 2-Ethoxy-4-fluorphenyl, 3-Methoxyphenyl oder Benzhydryl bedeutet, |

und ihre Salze hergestellt werden.

**4.** Verfahren nach Anspruch 1, dadurch gekennzeichnet, daß Verbindungen der Formel Ic

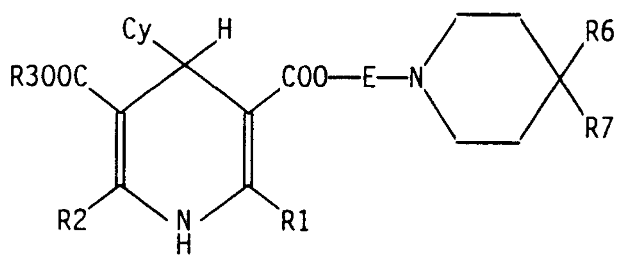

(Ic)

worin

| | |
|---|---|
| Cy | 3-Nitrophenyl oder 2,3-Dichlorphenyl bedeutet, |
| R1 | Methyl oder Ethyl bedeutet, |
| R2 | Methyl oder Ethyl bedeutet, |
| R3 | Methyl oder Ethyl bedeutet, |
| E | Ethylen oder Propylen bedeutet, |
| R6 | Phenyl bedeutet und |
| R7 | Phenyl bedeutet, |

und ihre Salze hergestellt werden.

**5.** Verfahren nach Anspruch 1 zur Herstellung des (-)-1,4-Dihydro-2,6-dimethyl-4-(3-nitrophenyl)-pyridin-3,5-dicarbonsäure-3-methyl-5-[3-(4,4-diphenylpiperidyl-1)-propyl]-esters der Formel I* und seiner Salze,

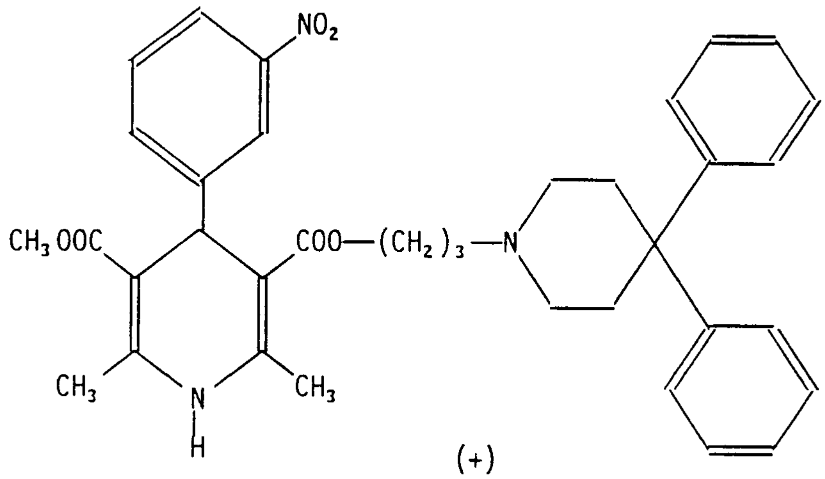

(+) (I*)

dadurch gekennzeichnet, daß man eine N-geschützte Dihydropyridincarbonsäure der Formel II*

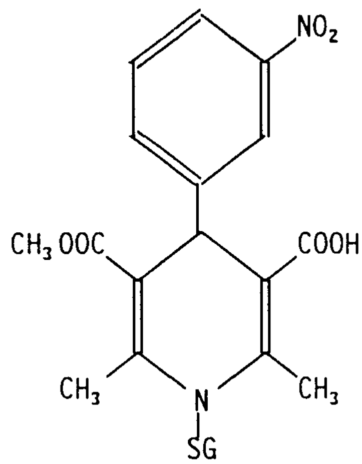

(II*) (+)

mit 1,3-Dibrompropan umsetzt und nach Abspaltung der Schutzgruppe SG den erhaltenen Brompropylester III*

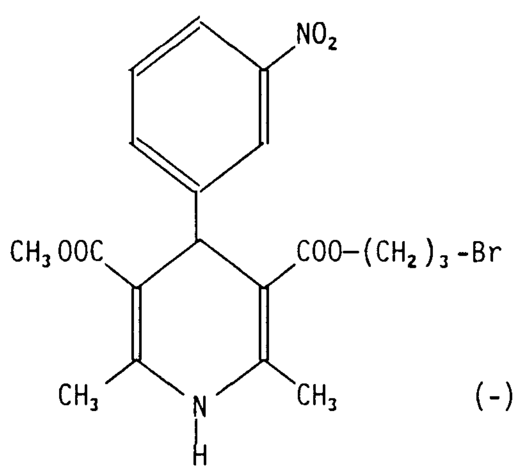

(III*) (-)

mit Diphenylpiperidin oder einem Salz davon umsetzt und gewünschtenfalls anschließend ein erhaltenes Salz der Verbindung I* in die freie Base oder die Verbindung I* in ein Salz überführt.

**6.** Verfahren nach Anspruch 1 oder 5, dadurch gekennzeichnet, daß die Schutzgruppe SG eine Alkoxymethylgruppe oder Benzyloxymethylgruppe ist.

## Claims

## Claims for the following Contracting States : AT, BE, CH, DE, FR, GB, IT, LI, LU, NL, SE

**1.** Pure dihydropyridine enantiomers of the formula I

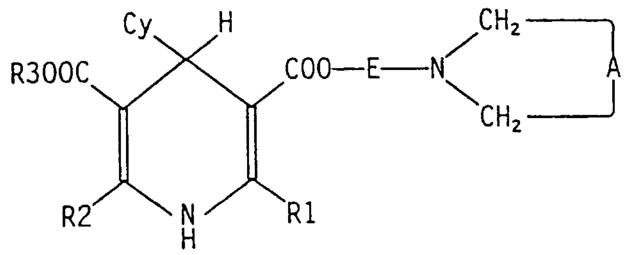

(I),

which, in the 4-position of the dihydropyridine, have the same configuration as the (+)-1-ethoxymethyl-1,4-dihydro-5-methoxycarbonyl-2,6-dimethyl-4-(3-nitrophenyl)-pyridine-3-carboxylic acid cinchonine salt which can be used as a precursor and which rotates linearly polarised light of wavelength 589 nm through $[\alpha]_D^{22}$ = + 101,5° (c = 1, chloroform),
and wherein

| | |
|---|---|
| Cy | denotes 3-nitrophenyl or 2,3-dichlorophenyl, |
| R1 and R2 | are identical or different and denote 1-4C-alkyl, |
| R3 | denotes 1-4C-alkyl, |
| E | denotes ethylene or propylene, |
| A | denotes $-CH_2-C(R6)R7-CH_2-$ or $-CH_2-NR8-CH_2-$, |
| R6 | denotes hydrogen (H) or phenyl, |
| R7 | denotes phenyl and |
| R8 | denotes aryl or benzhydryl, |

aryl representing a ring of the formula

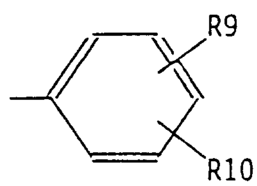

wherein R9 and R10 are identical or different and denote hydrogen (H), 1-4C-alkoxy or halogen, and the salts of these compounds.

**2.** Pure enantiomeric compounds according to claim 1, characterized by the formula Ia

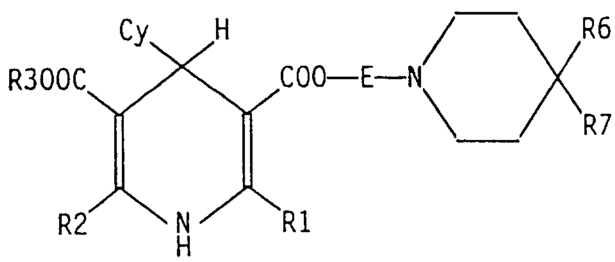

(Ia)

wherein

Cy denotes 3-nitrophenyl or 2,3-dichlorophenyl,
R1 denotes methyl or ethyl,
R2 denotes methyl or ethyl,
R3 denotes methyl or ethyl,
E denotes ethylene or propylene,
R6 denotes hydrogen and
R7 denotes phenyl
and their salts.

**3.** Pure enantiomeric compounds according to claim 1, characterized by the formula Ib

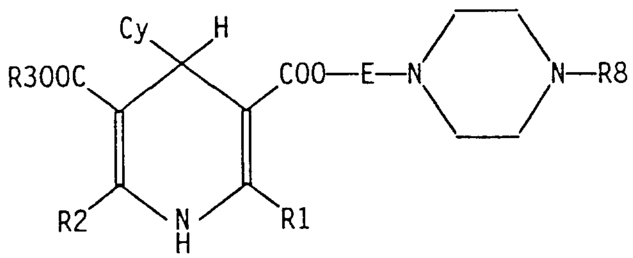
(Ib)

wherein
Cy denotes 3-nitrophenyl or 2,3-dichlorophenyl,
R1 denotes methyl or ethyl,
R2 denotes methyl or ethyl,
R3 denotes methyl or ethyl,
E denotes ethylene or propylene and
R8 denotes phenyl, 2-methoxyphenyl, 2-ethoxyphenyl, 2-ethoxy-4-fluorophenyl, 3-methoxyphenyl or benzhydryl,
and their salts.

**4.** Pure enantiomeric compounds according to claim 1, characterized by the formula Ic

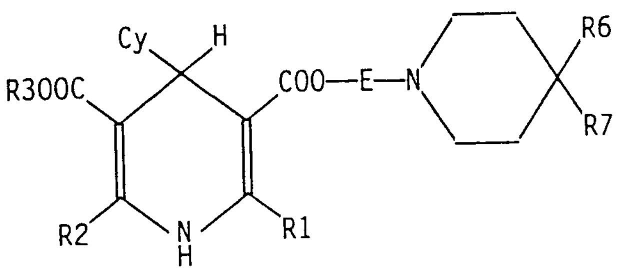
(Ic)

wherein
Cy denotes 3-nitrophenyl or 2,3-dichlorophenyl,
R1 denotes methyl or ethyl,
R2 denotes methyl or ethyl,
R3 denotes methyl or ethyl,
E denotes ethylene or propylene,
R6 denotes phenyl and
R7 denotes phenyl
and their salts.

**5.** 3-methyl-5-[3-(4,4-diphenylpiperid-1-yl)-propyl] (+)-1,4-dihydro-2,6-dimethyl-4-(3-nitrophenyl)-pyridine-3,5-dicarboxylate and its salts.

6. Process for the preparation of pure enantiomeric compounds of the formula I according to claim 1, and their salts, characterized in that pure enantiomeric N-protected dihydropyridinecarboxylic acids of the formula II

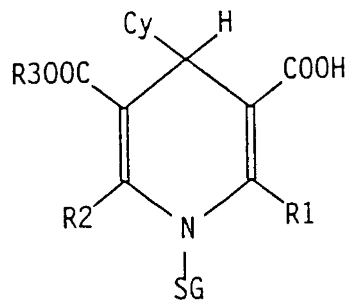

(II)

which, in the 4-position of the dihydropyridine, have the same configuration as the (+)-1-ethoxymethyl-1,4-dihydro-5-methoxycarbonyl-2,6-dimethyl-4-(3-nitrophenyl)-pyridine-3-carboxylic acid cinchonine salt which can be used as a precursor and which rotates linearly polarised light of wavelength 589 nm through $[\alpha]_D^{22}$ = +101,5° (c=1, chloroform)
and wherein R1, R3, R3 and Cy have the meanings given in claim 1 and SG represents a protective group, are reacted with an omega-dihaloalkane Hal-E-Hal, wherein Hal denotes halogen and E has the meaning given in claim 1, and, after elimination of the protective group SG, the resulting haloalkyl ester III

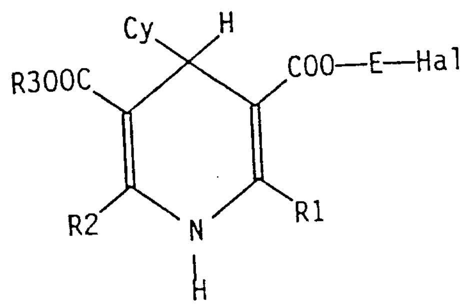

(III)

is reacted with an amine of the formula IV or a salt thereof

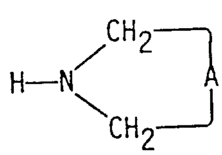

(IV)

wherein A has the meaning given in claim 1, and, if desired, salts of the compound I which are obtained are then converted into the free bases or the compounds I are converted into their salts.

7. Process according to claim 6 for the prepartion of 3-methyl 5-[3-(4,4-diphenylpiperid-1-yl)-propyl] (+)-1,4-dihydro-2,6-dimethyl-4-(3-nitrophenyl)-pyridine-3,5-dicarboxylate of the formula I* and its salts

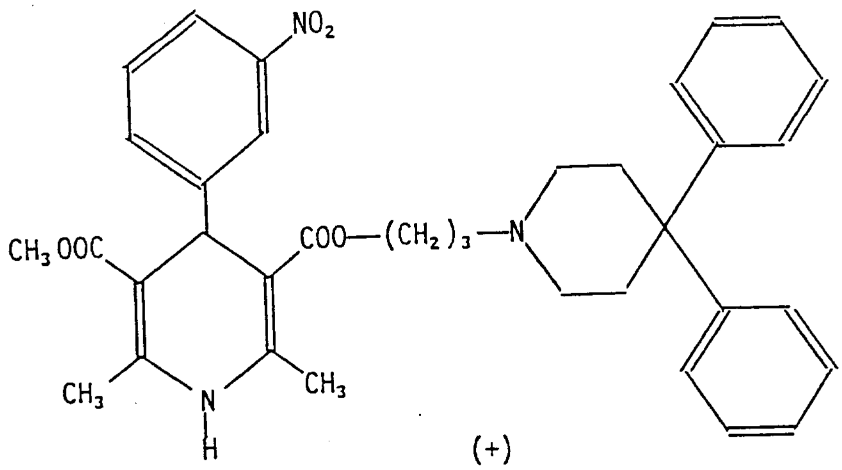

(I*)

(+)

characterized in that an N-protected dihydropyridinecarboxylic acid of the formula II*

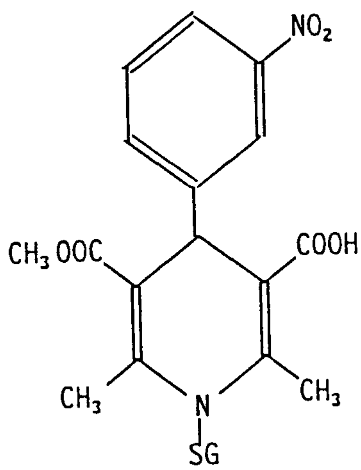

(II*)

(+)

is reacted with 1,3-dibromopropane and, after elimination of the protective group SG, the resulting bromopropyl ester III*

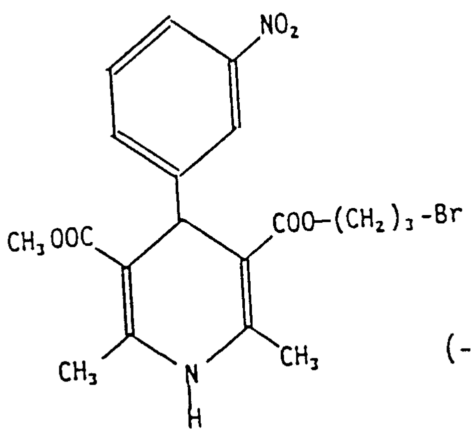

(III*)

(-)

is reacted with diphenylpiperidine or a salt thereof and, if desired, a resulting salt of the compound I* is then converted into the free base or the compound I* is converted into a salt.

**8.** Process according to claim 6 or 7, characterized in that the protective group SG is an alkoxymethyl group or a benzyloxymethyl group.

**9.** Medicament containing one or more compounds according to one or more of claims 1 to 5 and/or their pharmacologically acceptable salts.

**10.** Compounds according to one or more of claims 1 to 5 and their pharmacologically acceptable salts for use in the treatment and/or prophylaxis of arteriosclerosis.

**Claims for the following Contracting States : ES, GR**

**1.** Process for the preparation of pure dihydropyridine enantiomers of the formula I

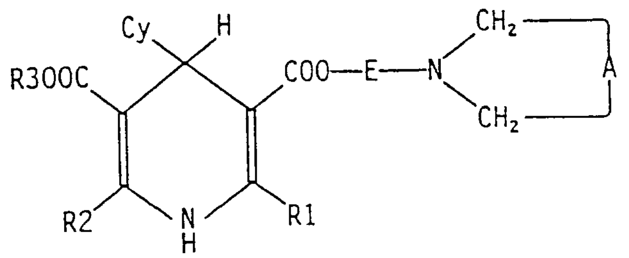

(I),

which, in the 4-position of the dihydropyridine, have the same configuration as the (+)-1-ethoxymethyl-1,4-dihydro-5-methoxycarbonyl-2,6-dimethyl-4-(3-nitrophenyl)-pyridine-3-carboxylic acid cinchonine salt which can be used as a precursor and which rotates linearly polarised light of wavelength 589 nm through $[\alpha]_D^{22}$ = + 101,5° (c = 1, chloroform),
and wherein

| | |
|---|---|
| Cy | denotes 3-nitrophenyl or 2,3-dichlorophenyl, |
| R1 and R2 | are identical or different and denote 1-4C-alkyl, |
| R3 | denotes 1-4C-alkyl, |
| E | denotes ethylene or propylene, |
| A | denotes $-CH_2-C(R6)R7-CH_2-$ or $-CH_2-NR8-CH_2-$, |
| R6 | denotes hydrogen (H) or phenyl, |
| R7 | denotes phenyl and |
| R8 | denotes aryl or benzhydryl, |

aryl representing a ring of the formula

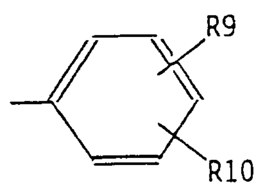

wherein R9 and R10 are identical or different and denote hydrogen (H), 1-4C-alkoxy or halogen,
and their salts, characterized in that pure enantiomeric N-protected dihydropyridinecarboxylic acids of the formula II

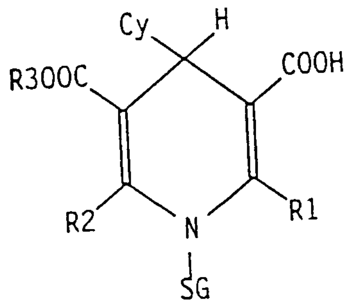

(II)

which, in the 4-position of the dihydropyridine, have the same configuration as the (+)-1-ethoxymethyl-1,4-dihydro-5-methoxycarbonyl-2,6-dimethyl-4-(3-nitrophenyl)-pyridine-3-carboxylic acid cinchonine salt which can be used as a precursor and which rotates linearly polarised light of wavelength 589 nm through $[\alpha]_D^{22}$ = +101,5° (c=1, chloroform)
and wherein R1, R2, R3 and Cy have the meanings given above and SG represents a protective group, are reacted with an omega-dihaloalkane Hal-E-Hal, wherein Hal denotes halogen and E has the meaning given above, and, after elimination of the protective group SG, the resulting haloalkyl ester III

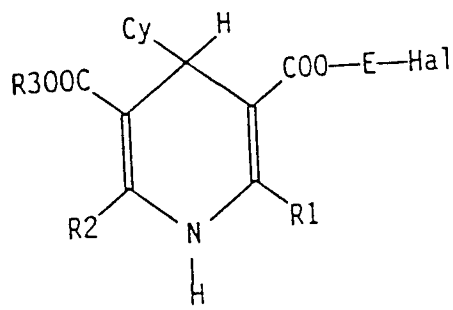

(III)

is reacted with an amine of the formula IV or a salt thereof

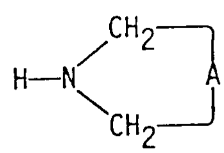

(IV)

wherein A has the abovementioned meaning, and, if desired, salts of the compound I which are obtained are then converted into the free bases or the compounds I are converted into their salts.

**2.** Process according to claim 1, characterized in that compounds of formula Ia

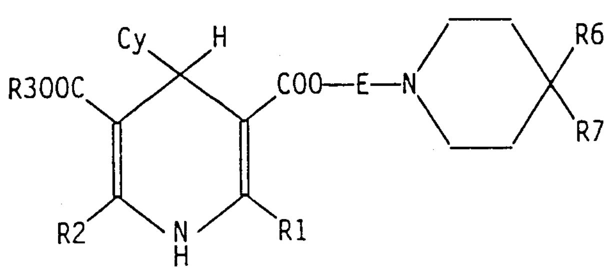

(Ia)

wherein

Cy denotes 3-nitrophenyl or 2,3-dichlorophenyl,
R1 denotes methyl or ethyl,
R2 denotes methyl or ethyl,
R3 denotes methyl or ethyl,
E denotes ethylene or propylene,
R6 denotes hydrogen and
R7 denotes phenyl
and their salts are prepared.

**3.** Process according to claim 1, characterized in that compounds of formula Ib

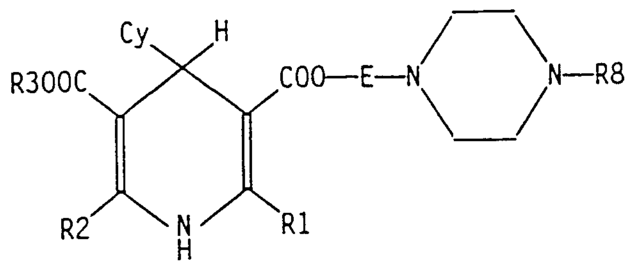

(Ib)

wherein
Cy denotes 3-nitrophenyl or 2,3-dichlorophenyl,
R1 denotes methyl or ethyl,
R2 denotes methyl or ethyl,
R3 denotes methyl or ethyl,
E denotes ethylene or propylene and
R8 denotes phenyl, 2-methoxyphenyl, 2-ethoxyphenyl, 2-ethoxy-4-fluorophenyl, 3-methoxyphenyl or benzhydryl,
and their salts are prepared.

**4.** Process according to claim 1, characterized in that compounds of formula Ic

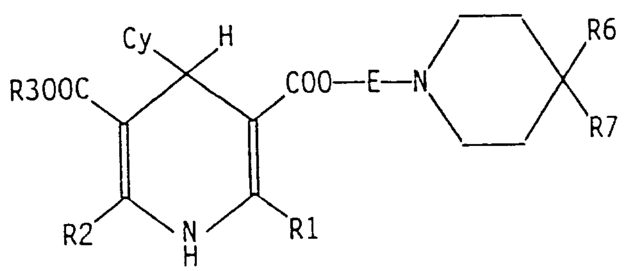

(Ic)

wherein
Cy denotes 3-nitrophenyl or 2,3-dichlorophenyl,
R1 denotes methyl or ethyl,
R2 denotes methyl or ethyl,
R3 denotes methyl or ethyl,
E denotes ethylene or propylene,
R6 denotes phenyl and
R7 denotes phenyl
and their salts are prepared.

**5.** Process according to claim 1 for the prepartion of 3-methyl 5-[3-(4,4-diphenylpiperid-1-yl)-propyl] (+)-1,4-dihydro-2,6-dimethyl-4-(3-nitrophenyl)-pyridine-3,5-dicarboxylate of the formula I* and its salts

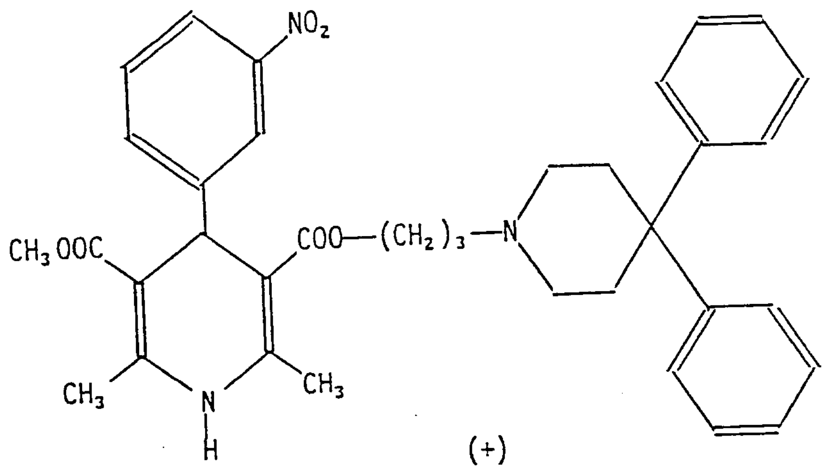

(I*)

(+)

characterized in that an N-protected dihydropyridinecarboxylic acid of the formula II*

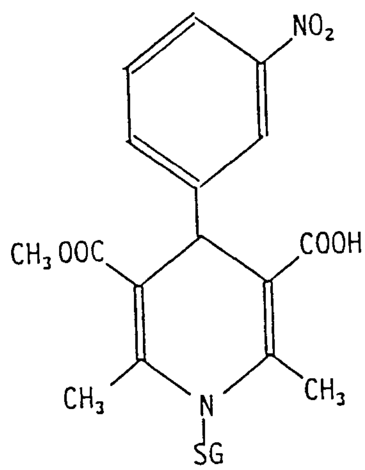

(II*)

(÷)

is reacted with 1,3-dibromopropane and, after elimination of the protective group SG, the resulting bromopropyl ester III*

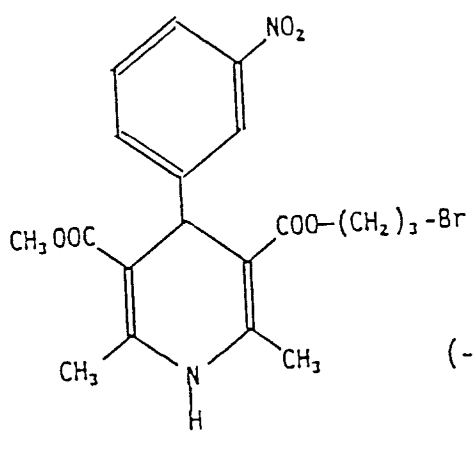

(III*)

(-)

is reacted with diphenylpiperidine or a salt thereof and, if desired, a resulting salt of the compound I* is then converted into the free base or the compound I* is converted into a salt.

6. Process according to claim 1 or 5, characterized in that the protective group SG is an alkoxymethyl group or a benzyloxymethyl group.

**Revendications**

**Revendications pour les Etats contractants suivants : AT, BE, CH, DE, FR, GB, IT, LI, LU, NL, SE**

1. Dihydropyridines énantiomères pures de formule I :

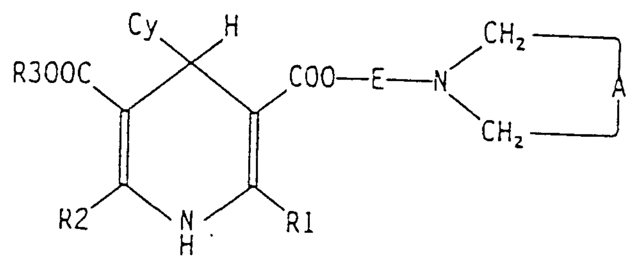

(I),

qui présentent dans la position 4 de la dihydropyridine la même configuration que le (+)-1-éthoxyméthyl-1,4-dihydro-5-méthoxycarbonyl-2,6-diméthyl-4-(3-nitrophényl)-pyridine-3-carboxylate de cinchonine utilisable comme produit intermédiaire, qui fait tourner le plan de polarisation de la lumière polarisée linéairement de longueur d'onde 589 nm avec une rotation spécifique $[\alpha]_D^{22}$ = + 101,5° (c = 1, chloroforme)
et dans laquelle
Cy représente un groupe 3-nitrophényle ou 2,3-dichlorophényle,
R1 et R2 sont identiques ou différents et représentent alkyle en $C_1$-$C_4$,
R3 représente un groupe alkyle en $C_1$-$C_4$,
E représente un groupe éthylène ou propylène,
A représente un groupe $-CH_2-C(R6)R7-CH_2-$ ou $-CH_2-NR8-CH_2-$,
R6 représente l'hydrogène (H) ou un groupe phényle,
R7 représente un groupe phényle,
R8 représente un groupe aryle ou benzhydryle,
aryle étant un noyau de formule :

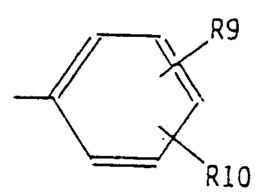

dans laquelle
R9 et R10 sont identiques ou différents et représentent l'hydrogène (H) ou un halogène ou un groupe alcoxy en $C_1$-$C_4$
et les sels de ces composés.

2. Composés énantiomères purs selon la revendication 1, caractérisés par la formule Ia :

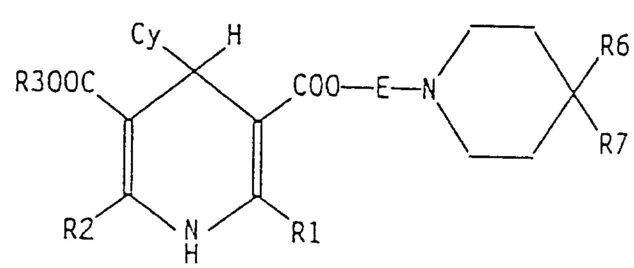

(Ia)

dans laquelle
Cy représente un groupe 3-nitrophényle ou 2,3-dichlorophényle,
R1 représente un groupe méthyle ou éthyle,
R2 représente un groupe méthyle ou éthyle,
R3 représente un groupe méthyle ou éthyle,
E représente un groupe éthylène ou propylène,
R6 représente l'hydrogène et
R7 représente un groupe phényle
et leurs sels.

3. Composés énantiomères purs selon la revendication 1, caractérisés par la formule Ib :

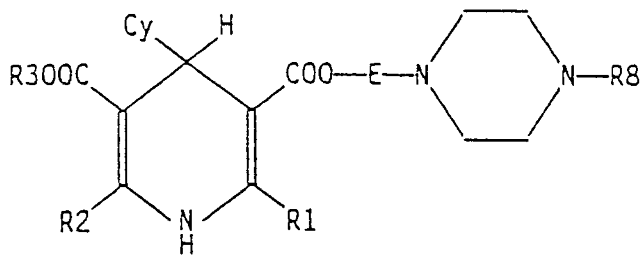

(Ib)

dans laquelle
Cy représente un groupe 3-nitrophényle ou 2,3-dichlorophényle,
R1 représente un groupe méthyle ou éthyle,
R2 représente un groupe méthyle ou éthyle,
R3 représente un groupe méthyle ou éthyle,
E représente un groupe éthylène ou propylène et
R8 représente un groupe phényle, 2-méthoxyphényle, 2-éthoxyphényle, 2-éthoxy-4-fluorophényle, 3-méthoxyphényle ou benzhydryle,
et leurs sels.

4. Composés énantiomères purs selon la revendication 1, caractérisés par la formule Ic :

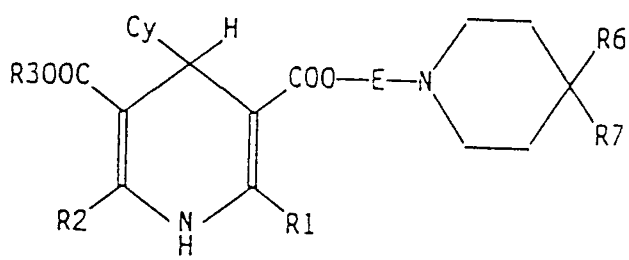

(Ic)

dans laquelle
Cy représente un groupe 3-nitrophényle ou 2,3-dichlorophényle,
R1 représente un groupe méthyle ou éthyle,
R2 représente un groupe méthyle ou éthyle,
R3 représente un groupe méthyle ou éthyle,
E représente un groupe éthylène ou propylène,
R6 représente un groupe phényle et
R7 représente un groupe phényle
et leurs sels.

5. Le (+)-1,4-dihydro-2,6-diméthyl-4-(3-nitrophényl)-pyridine-3,5-dicarboxylate de 3-méthyle et de 5-[3-(4,4-diphénylpipéridyl-1)-propyle] et ses sels.

6. Procédé pour la fabrication des composés énantiomères purs de formule I selon la revendication 1 et de leurs sels, caractérisé en ce que l'on fait réagir des acides dihydropyridinecarboxyliques énantiomères purs N-protégés de formule II :

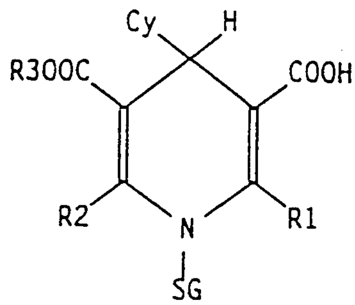

(II)

qui présentent dans la position 4 de la dihydropyridine la même configuration que le (+)-1-éthoxyméthyl-1,4-dihydro-5-méthoxycarbonyl-2,6-diméthyl-4-(3-nitrophényl)-pyridine-3-carboxylate de cinchonine utilisable comme produit intermédiaire, qui fait tourner le plan de polarisation de la lumière polarisée linéairement de longueur d'onde 589 nm avec une rotation spécifique $[\alpha]_D^{22}$ = + 101,5° (c = 1, chloroforme) et dans laquelle R1, R2, R3 et Cy ont les significations indiquées dans la revendication 1 et SG représente un groupe protecteur, avec un ω-dihalogénoalcane Hal-E-Hal, dans lequel Hal représente un halogène et E a la signification indiquée dans la revendication 1, et après élimination du groupe protecteur SG, on fait réagir l'ester d'halogénoalkyle III obtenu :

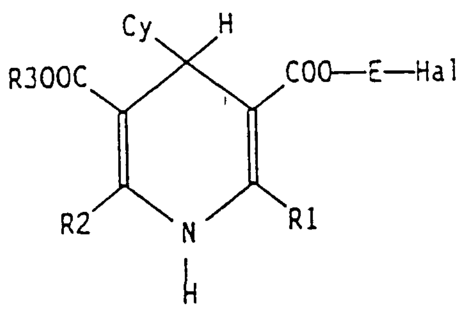

(III)

avec une amine de formule IV :

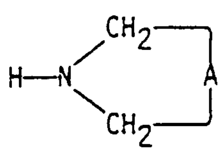

(IV)

dans laquelle A a la signification indiquée dans la revendication 1, ou un de ses sels et si on le désire, on transforme ensuite les sels des composés I obtenus en les bases libres ou les composés I en leurs sels.

7. Procédé selon la revendication 6, pour la fabrication du (+)-1,4-dihydro-2,6-diméthyl-4-(3-nitrophényl)-pyridlne-3,5-dicarboxylate de 3-méthyle et de 5-[3-(4,4-diphénylpipéridyl-1)-propyle] de formule I* :

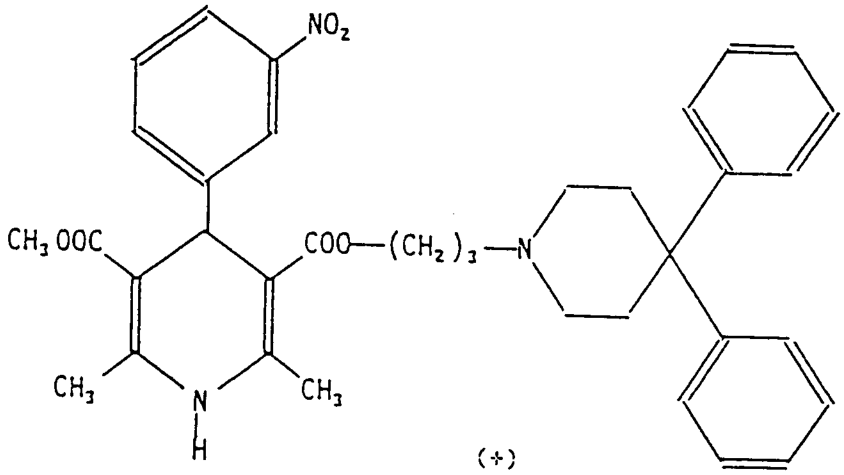

(+) (I*)

et de ses sels caractérisé en ce que l'on fait réagir un acide dihydropyridinecarboxylique N-protégé de formule II* :

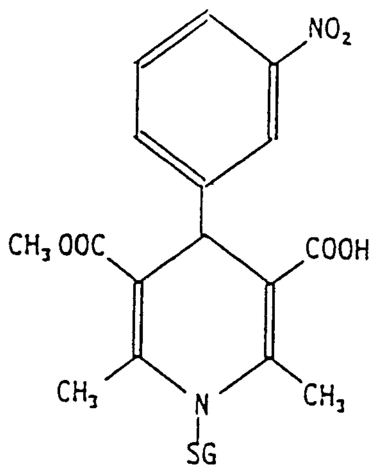

(II*) (+)

avec le 1,3-dibromopropane et après élimination du groupe protecteur SG on fait réagir l'ester de bromopropyle III* obtenu:

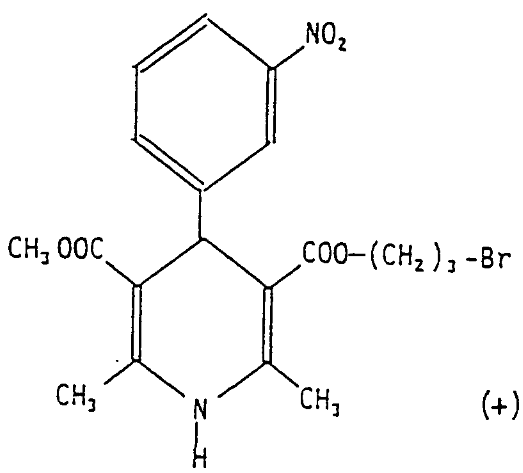

(III*) (+)

avec la diphénylpipéridine ou un de ses sels et, si on le désire, on transforme ensuite un sel du

composé I* obtenu en la base libre ou le composé I* en un sel.

8. Procédé selon la revendication 6 ou 7, caractérisé en ce que le groupe protecteur SG est un groupe alcoxyméthyle ou un groupe benzyloxyméthyle.

9. Médicament contenant un ou plusieurs composés selon une ou plusieurs des revendications 1 à 5 et/ou leurs sels pharmacologiquement acceptables.

10. Composés selon une ou plusieurs des revendications 1 à 5 et leurs sels pharmacologiquement acceptables pour l'application au traitement et/ou à la prophylaxie de l'artériosclérose.

**Revendications pour les Etats contractants suivants : ES, GR**

1. Procédé pour la fabrication de dihydropyridines énantiomères pures de formule I :

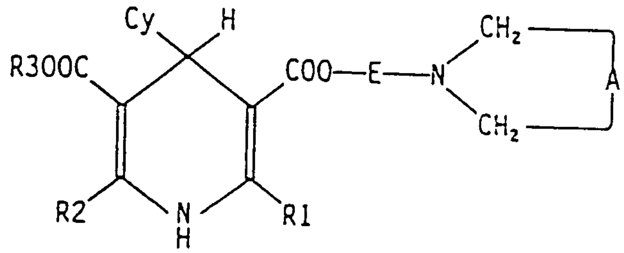

(I),

qui présentent dans la position 4 de la dihydropyridine la même configuration que le (+)-1-éthoxyméthyl-1,4-dihydro-5-méthoxycarbonyl-2,6-diméthyl-4-(3-nitrophényl)-pyridine-3-carboxylate de cinchonine utilisable comme produit intermédiaire, qui fait tourner le plan de polarisation de la lumière polarisée linéairement de longueur d'onde 589 nm avec une rotation spécifique $[\alpha]_D^{22}$ = + 101,5° (c = 1, chloroforme)
et dans laquelle
Cy représente un groupe 3-nitrophényle ou 2,3-dichlorophényle,
R1 et R2 sont identiques ou différents et représentent alkyle en $C_1$-$C_4$,
R3 représente un groupe alkyle en $C_1$-$C_4$,
E représente un groupe éthylène ou propylène,
A représente un groupe $-CH_2-C(R6)R7-CH_2-$ ou $-CH_2-NR8-CH_2-$,
R6 représente l'hydrogène (H) ou un groupe phényle,
R7 représente un groupe phényle,
R8 représente un groupe aryle ou benzhydryle,
aryle étant un noyau de formule :

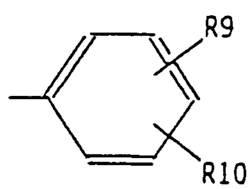

dans laquelle
R9 et R10 sont identiques ou différents et représentent l'hydrogène (H) ou un halogène ou un groupe alcoxy en $C_1$-$C_4$
et de leurs sels, caractérisé en ce que l'on fait réagir des acides dihydropyridinecarboxyliques énantiomères purs N-protégés de formule II :

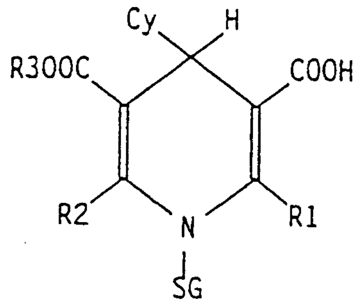

(II)

qui présentent dans la position 4 de la dihydropyridine la même configuration que le (+)-1-éthoxyméthyl-1,4-dihydro-5-méthoxycarbonyl-2,6-diméthyl-4-(3-nitrophényl)-pyridine-3-carboxylate de cinchonine utilisable comme produit intermédiaire, qui fait tourner le plan de polarisation de la lumière polarisée linéairement de longueur d'onde 589 nm avec une rotation spécifique $[\alpha]_D^{22}$ = + 101,5° (c = 1, chloroforme) et dans laquelle R1, R2, R3 et Cy ont les significations indiquées ci-dessus et SG représente un groupe protecteur, avec un ω-dihalogénoalcane Hal-E-Hal, dans lequel Hal représente un halogène et E a la signification indiquée ci-dessus, et après élimination du groupe protecteur SG, on fait réagir l'ester d'halogénoalkyle III obtenu :

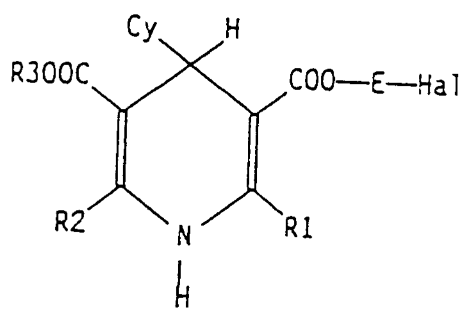

(III)

avec une amine de formule IV :

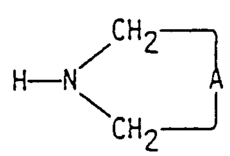

(IV)

dans laquelle A a la signification indiquée ci-dessus ou un de ses sels, et si on le désire, on transforme ensuite les sels des composés I obtenus en les bases libres ou les composés I en leurs sels.

2. Procédé selon la revendication 1, caractérisé en ce que l'on fabrique des composés de formule Ia :

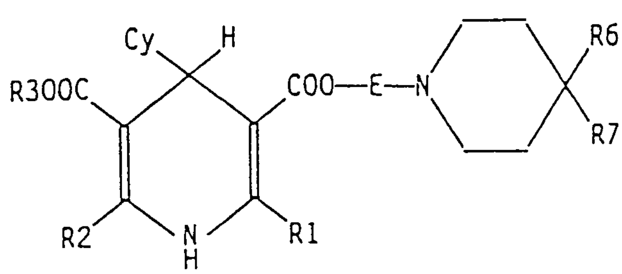

(Ia)

dans laquelle

Cy représente un groupe 3-nitrophényle ou 2,3-dichlorophényle,
R1 représente un groupe méthyle ou éthyle,
R2 représente un groupe méthyle ou éthyle,
R3 représente un groupe méthyle ou éthyle,
E représente un groupe éthylène ou propylène,
R6 représente l'hydrogène et
R7 représente un groupe phényle
et leurs sels.

**3.** Procédé selon la revendication 1, caractérisé en ce que l'on fabrique des composés de formule Ib :

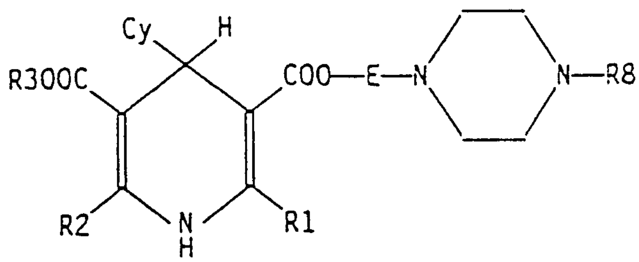

(Ib)

dans laquelle
Cy représente un groupe 3-nitrophényle ou 2,3-dichlorophényle,
R1 représente un groupe méthyle ou éthyle,
R2 représente un groupe méthyle ou éthyle,
R3 représente un groupe méthyle ou éthyle,
E représente un groupe éthylène ou propylène, et
R8 représente un groupe phényle, 2-méthoxyphényle, 2-éthoxyphényle, 2-éthoxy-4-fluorophényle, 3-méthoxyphényle ou benzhydryle,
et leurs sels.

**4.** Procédé selon la revendication 1, caractérisé en ce que l'on fabrique des composés de formule Ic :

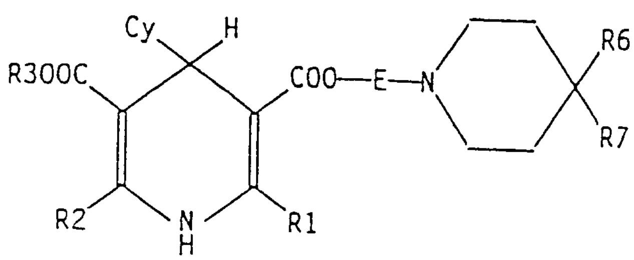

(Ic)

dans laquelle
Cy représente un groupe 3-nitrophényle ou 2,3-dichlorophényle,
R1 représente un groupe méthyle ou éthyle,
R2 représente un groupe méthyle ou éthyle,
R3 représente un groupe méthyle ou éthyle,
E représente un groupe éthylène ou propylène,
R6 représente un groupe phényle et
R7 représente un groupe phényle
et leurs sels.

**5.** Procédé selon la revendication 1, pour la fabrication du (+)-1,4-dihydro-2,6-diméthyl-4-(3-nitrophényl)-pyridine-3,5-dicarboxylate de 3-méthyle et de 5-[3-(4,4-diphénylpipéridyl-1)-propyle] de formule I* :

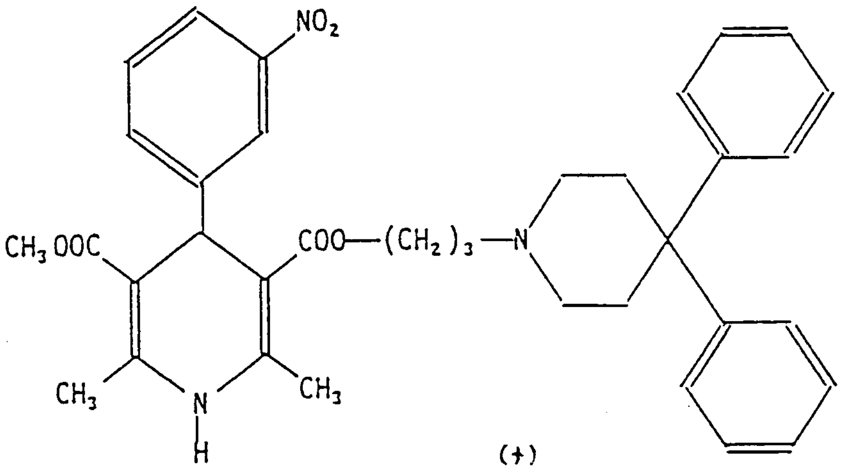

(+)

(I*)

et de ses sels caractérisé en ce que l'on fait réagir un acide dihydropyridinecarboxylique N-protégé de formule II* :

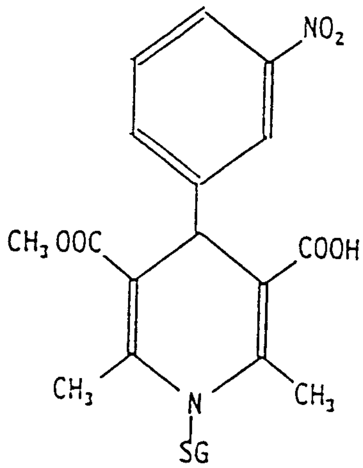

(II*)

(+)

avec le 1,3-dibromopropane et après élimination du groupe protecteur SG on fait réagir l'ester de bromopropyle III* obtenu :

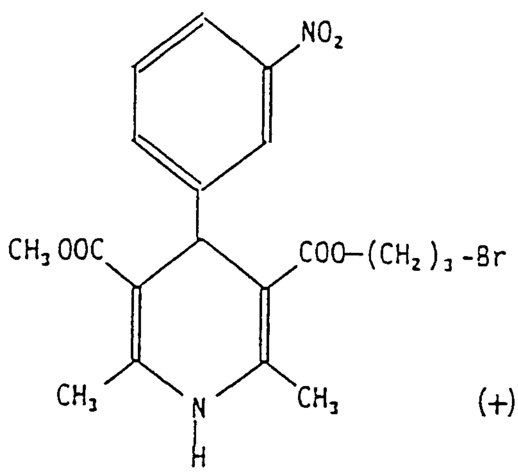

(III*)

(+)

avec la diphénylpipéridine ou un de ses sels et si on le désire, on transforme ensuite un sel du composé I* obtenu en la base libre ou le composé I* en un sel.

6. Procédé selon la revendication 1 ou 5, caractérisé en ce que le groupe protecteur SG est un groupe alcoxyméthyle ou un groupe benzyloxyméthyle.